(19) Europäisches Patentamt
European Patent Office
Office européen des brevets

(11) **EP 1 221 845 B1**

(12) **EUROPÄISCHE PATENTSCHRIFT**

(45) Veröffentlichungstag und Bekanntmachung des Hinweises auf die Patenterteilung:
**25.06.2003 Patentblatt 2003/26**

(21) Anmeldenummer: **00967765.9**

(22) Anmeldetag: **25.09.2000**

(51) Int Cl.⁷: **A01N 43/12**
// (A01N43/12, 51:00), A01N47:40

(86) Internationale Anmeldenummer:
**PCT/EP00/09323**

(87) Internationale Veröffentlichungsnummer:
**WO 01/024634 (12.04.2001 Gazette 2001/15)**

(54) **WIRKSTOFFKOMBINATIONEN MIT INSEKTIZIDEN UND AKARIZIDEN EIGENSCHAFTEN**

ACTIVE INGREDIENT COMBINATIONS HAVING INSECTICIDAL AND ACARICIDAL PROPERTIES

COMBINAISONS D'AGENTS ACTIFS PRESENTANT DES PROPRIETES ACARICIDES ET INSECTICIDES

(84) Benannte Vertragsstaaten:
**AT BE CH CY DE DK ES FI FR GB GR IE IT LI LU MC NL PT SE**

(30) Priorität: **07.10.1999 DE 19948129**

(43) Veröffentlichungstag der Anmeldung:
**17.07.2002 Patentblatt 2002/29**

(73) Patentinhaber: **Bayer CropScience AG**
**40789 Monheim (DE)**

(72) Erfinder:
- **FISCHER, Reiner**
  **40789 Monheim (DE)**
- **ERDELEN, Christoph**
  **42799 Leichlingen (DE)**

(56) Entgegenhaltungen:
**EP-A- 0 528 156**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue Wirkstoffkombinationen, die aus einem bekannten cyclischen Ketoenol einerseits und weiteren bekannten insektiziden Wirkstoffen andererseits bestehen und sehr gute insektizide und akarizide Eigenschaften besitzen.

Es ist bereits bekannt, dass bestimmte cyclische Ketoenole zur Bekämpfung von tierischen Schädlingen, wie Insekten und unerwünschten Akariden eingesetzt werden können (vgl. EP-A-528 156). Die Wirksamkeit dieser Stoffe ist gut, lässt aber bei niedrigen Aufwandmengen in manchen Fällen zu wünschen übrig.

Desweiteren ist auch bekannt geworden, dass man Agonisten und Antagonisten von nicotinergen Acetylcholinrezeptoren zur Bekämpfung von Insekten verwenden kann.

Es wurde nun gefunden, dass Mischungen aus cyclischen Ketoenolen der Formel (I)

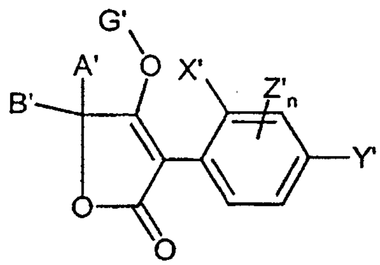

(I)

in welcher

X' für $C_1$-$C_6$-Alkyl, Halogen, $C_1$-$C_6$-Alkoxy oder $C_1$-$C_3$-Halogenalkyl steht,

Y' für Wasserstoff, $C_1$-$C_6$-Alkyl, Halogen, $C_1$-$C_6$-Alkoxy, $C_1$-$C_3$-Halogenalkyl steht,

Z' für $C_1$-$C_6$-Alkyl, Halogen, $C_1$-$C_6$-Alkoxy steht,

n für eine Zahl von 0-3 steht,

A' und B' gleich oder verschieden sind und fiir Wasserstoff oder gegebenenfalls durch Halogen substituiertes geradkettiges oder verzweigtes $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_2$-$C_8$-alkyl, $C_1$-$C_{10}$-Alkylthio-$C_2$-$C_8$-alkyl, Cycloalkyl mit 3-8 Ringatomen, das durch Sauerstoff und/oder Schwefel unterbrochen sein kann und gegebenenfalls durch Halogen, $C_1$-$C_6$-Alkyl, $C_1$-$C_6$-Halogenalkyl-, $C_1$-$C_6$-Alkoxy-, $C_1$-$C_6$-Halogenalkoxy, Nitro substituiertes Phenyl oder Phenyl-$C_1$-$C_6$-alkyl steht,

oder worin

A' und B' gemeinsam mit dem Kohlenstoffatom, an das sie gebunden sind einen gesättigten oder ungesättigten, gegebenenfalls durch Sauerstoff und/oder Schwefel unterbrochenen und gegebenenfalls durch Halogen, $C_1$-$C_6$-Alkyl, $C_1$-$C_6$-Alkoxy, $C_1$-$C_4$-Halogenalkyl, $C_1$-$C_4$-Halogenalkoxy, $C_1$-$C_4$-Alkylthio oder gegebenenfalls substituiertes Phenyl substituierten oder gegebenenfalls benzokondensierten 3- bis 8-gliedrigen Ring bilden,

G' für Wasserstoff (a) oder für die Gruppen

$-CO\text{-}R^1$ (b), 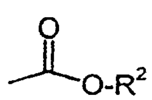 (c), $-SO_2\text{-}R^3$ (d),

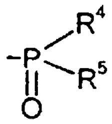 (e) oder 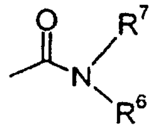 (f)

steht, in welchen

R¹ für gegebenenfalls durch Halogen substituiertes: $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Alkylthio-$C_2$-$C_8$-alkyl, $C_1$-$C_8$-Polyalkoxy-$C_2$-$C_8$-alkyl oder Cycloalkyl mit 3-8 Ringatomen, das durch Sauerstoff- und/oder Schwefelatome unterbrochen sein kann, steht,
für gegebenenfalls durch Halogen, Nitro, $C_1$-$C_6$-Alkyl, $C_1$-$C_6$-Alkoxy, $C_1$-$C_6$-Halogenalkyl, $C_1$-$C_6$-Halogenalkoxy-substituiertes Phenyl steht;
für gegebenenfalls durch Halogen-, $C_1$-$C_6$-Alkyl, $C_1$-$C_6$-Alkoxy-, $C_1$-$C_6$-Halogenalkyl-, $C_1$-$C_6$-Halogenalkoxy-substituiertes Phenyl-$C_1$-$C_6$-alkyl steht,
für gegebenenfalls durch Halogen und/oder $C_1$-$C_6$-Alkyl substituiertes Pyridyl, Pyrimidyl, Thiazolyl und Pyrazolyl steht,
für gegebenenfalls durch Halogen und/oder $C_1$-$C_6$-Alkyl-substituiertes Phenoxy-$C_1$-$C_6$-alkyl steht,

R² für gegebenenfalls durch Halogen substituiertes: $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Polyalkoxy-$C_2$-$C_8$-alkyl steht,
für gegebenenfalls durch Halogen, Nitro, $C_1$-$C_6$-Alkyl, $C_1$-$C_6$-Alkoxy, $C_1$-$C_6$-Halogenalkyl-substituiertes Phenyl oder Benzyl steht,

$R^3$, $R^4$ und $R^5$ unabhängig voneinander für gegebenenfalls durch Halogen substituiertes $C_1$-$C_8$-Alkyl, $C_1$-$C_8$-Alkoxy, $C_1$-$C_8$-Alkylamino, Di-($C_1$-$C_8$)-Alkylamino, $C_1$-$C_8$-Alkylthio, $C_2$-$C_5$-Alkenylthio, $C_2$-$C_5$-Alkinylthio, $C_3$-$C_7$-Cycloalkylthio, für gegebenenfalls durch Halogen, Nitro, Cyano, $C_1$-$C_4$-Alkoxy, $C_1$-$C_4$-Halogenalkoxy, $C_1$-$C_4$-Alkylthio, $C_1$-$C_4$-Halogen-alkylthio, $C_1$-$C_4$-Alkyl, $C_1$-$C_4$-Halogenalkyl substituiertes Phenyl, Phenoxy oder Phenylthio stehen,

$R^6$ und $R^7$ unabhängig voneinander für gegebenenfalls durch Halogen substituiertes $C_1$-$C_{20}$-Alkyl, $C_1$-$C_{20}$-Alkoxy, $C_2$-$C_8$-Alkenyl, $C_1$-$C_{20}$-Alkoxy-$C_1$-$C_{20}$-alkyl, für gegebenenfalls durch Halogen, $C_1$-$C_{20}$-Halogenalkyl, $C_1$-$C_{20}$-Alkyl oder $C_1$-$C_{20}$-Alkoxy substituiertes Phenyl, fiir gegebenenfalls durch Halogen, $C_1$-$C_{20}$-Alkyl, $C_1$-$C_{20}$-Halogenalkyl oder $C_1$-$C_{20}$-Alkoxy substituiertes Benzyl steht oder zusammen für einen gegebenenfalls durch Sauerstoff unterbrochenen $C_2$-$C_6$-Alkylenring stehen,

und mindestens einem Agonisten bzw. Antagonisten von Acetylcholinrezeptoren der Formel (II) synergistisch wirksam sind und sich zur Bekämpfung tierischer Schädlinge eignen. Aufgrund dieses Synergismus können deutlich geringere Wirkstoffmengen verwendet werden, d.h. die Wirkung der Mischung ist größer als die Wirkung der Einzelkomponenten.

Bevorzugt sind Mischungen enthaltend Verbindungen der Formel (I)
in welcher

X' für $C_1$-$C_4$-Alkyl, Halogen, $C_1$-$C_4$-Alkoxy oder $C_1$-$C_2$-Halogenalkyl steht,
Y' für Wasserstoff, $C_1$-$C_4$-Alkyl, Halogen, $C_1$-$C_4$-Alkoxy, $C_1$-$C_2$-Halogenalkyl steht,
Z' für $C_1$-$C_4$-Alkyl, Halogen, $C_1$-$C_4$-Alkoxy steht,
n für 0 oder 1 steht,
A' und B' gemeinsam mit dem Kohlenstoffatom, an das sie gebunden sind einen gesättigten gegebenenfalls durch $C_1$-$C_4$-Alkyl, $C_1$-$C_4$-Alkoxy, substituierten 5- bis 6-gliedrigen Ring bilden,
G' für Wasserstoff (a) oder für die Gruppen

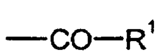 (b) 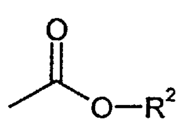 (c)

in welchen

R¹ für gegebenenfalls durch Halogen substituiertes: $C_1$-$C_{16}$-Alkyl, $C_2$-$C_{16}$-Alkenyl, $C_1$-$C_6$-Alkoxy-$C_2$-$C_6$-alkyl, oder Cycloalkyl mit 3-7 Ringatomen, das durch 1 bis 2 Sauerstoff- und/oder Schwefelatome unterbrochen

sein kann, steht,
für gegebenenfalls durch Halogen, Nitro, $C_1$-$C_4$-Alkyl, $C_1$-$C_4$-Alkoxy, $C_1$-$C_3$-Halogenalkyl, $C_1$-$C_3$-Halogenalkoxy-substituiertes Phenyl steht;

$R^2$ für gegebenenfalls durch Halogen substituiertes: $C_1$-$C_{16}$-Alkyl, $C_2$-$C_{16}$-Alkenyl, $C_1$-$C_6$-Alkoxy-$C_2$-$C_6$-alkyl, steht,
für gegebenenfalls durch Halogen, Nitro, $C_1$-$C_4$-Alkyl, $C_1$-$C_4$-Alkoxy, $C_1$-$C_4$-Halogenalkyl-substituiertes Phenyl oder Benzyl steht,

und mindestens einen Agonisten bzw. Antagonisten von Acetylcholinrezeptoren der Formel (II).

Besonders bevorzugt sind Mischungen enthaltend das Dihydrofuranonderivat der Formel (Ia)

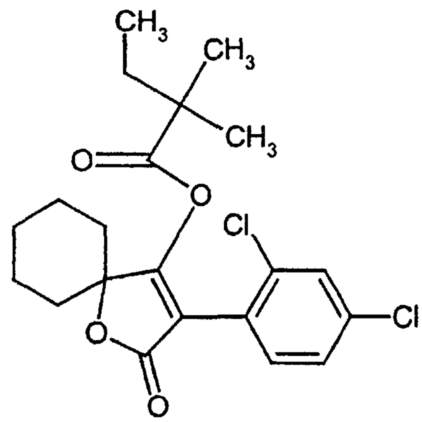

(Ia)

und mindestens einen Agonisten bzw. Antagonisten von Acetylcholinrezeptoren der Formel (II).

Bei den Agonisten und Antagonisten der nicotinergen Acetylcholinrezeptoren handelt es sich um bekannte Verbindungen, die bekannt sind aus folgenden Publikationen:

Europäische Offenlegungsschriften Nr. 464 830, 428 941, 425 978, 386 565, 383 091, 375 907, 364 844, 315 826, 259 738, 254 859, 235 725, 212 600, 192 060, 163 855, 154 178, 136 636, 136 686, 303 570, 302 833, 306 696, 189 972, 455 000, 135 956, 471 372, 302 389, 428 941, 376 279, 493 369, 580 553, 649 845, 685 477, 483 055, 580 553;

Deutsche Offenlegungsschriften Nr. 3 639 877, 3 712 307;

Japanische Offenlegungsschriften Nr. 03 220 176, 02 207 083, 63 307 857, 63 287 764, 03 246 283, 04 9371, 03 279 359, 03 255 072, 05 178 833, 07 173 157, 08 291 171;

US-Patentschriften Nr. 5 034 524, 4 948 798, 4 918 086, 5 039 686, 5 034 404, 5 532 365;

PCT-Anmeldungen Nr. WO 91/17 659, 91/4965;

Französische Anmeldung Nr. 2 611 114;

Brasilianische Anmeldung Nr. 88 03 621.

Auf die in diesen Publikationen beschriebenen generischen Formeln und Definitionen sowie auf die darin beschriebenen einzelnen Verbindungen wird hiermit ausdrücklich Bezug genommen.

Diese Verbindungen werden zum Teil unter dem Begriff Nitromethylene, Nitroimine und damit verwandte Verbindungen zusammengefasst.

Diese Verbindungen lassen sich bevorzugt unter der Formel (II) zusammenfassen

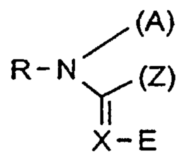

(II)

in welcher

R für Wasserstoff, gegebenenfalls substituierte Reste Acyl, Alkyl, Aryl, Aralkyl, Heterocyclyl, Heteroaryl oder Heteroarylalkyl steht;

A für eine monofunktionelle Gruppe aus der Reihe Wasserstoff, Acyl, Alkyl, Aryl steht oder für eine bifunktionelle Gruppe steht, die mit dem Rest Z verknüpft ist;

E für einen elektronenziehenden Rest steht;

X für die Reste -CH= oder =N- steht, wobei der Rest -CH= anstelle eines H-Atoms mit dem Rest Z verknüpft sein kann;

Z für eine monofunktionelle Gruppe aus der Reihe Alkyl, -O-R, -S-R,

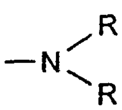

steht,
wobei die Reste R gleich oder verschieden sind und die oben angegebene Bedeutung haben,
oder für eine bifunktionelle Gruppe steht, die mit dem Rest A oder dem Rest X verknüpft ist.

Besonders bevorzugt sind Verbindungen der Formel (II), in welcher die Reste folgende Bedeutung haben:

R steht für Wasserstoff sowie für gegebenenfalls substituierte Reste aus der Reihe Acyl, Alkyl, Aryl, Aralkyl, Heterocyclylalkyl, Heteroaryl, Heteroarylalkyl.
Als Acylreste seien genannt Formyl, Alkylcarbonyl, Arylcarbonyl, Alkylsulfonyl, Arylsulfonyl, (Alkyl-)-(Aryl-)-phosphoryl, die ihrerseits substituiert sein können.
Als Alkyl sei genannt $C_1$-$C_{10}$-Alkyl, insbesondere $C_1$-$C_4$-Alkyl, im einzelnen Methyl, Ethyl, i-Propyl, sec.- oder t.-Butyl, die ihrerseits substituiert sein können.
Als Aryl sei genannt Phenyl, Naphthyl, insbesondere Phenyl.
Als Aralkyl sei genannt Phenylmethyl, Phenethyl.
Als Heterocyclylalkyl sei der Rest

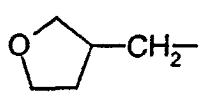

genannt.
Als Heteroaryl sei genannt Heteroaryl mit bis zu 10 Ringatomen und N, O, S, insbesondere N als Heteroatomen. Im einzelnen seien genannt Thienyl, Furyl, Thiazolyl, Imidazolyl, Pyridyl, Benzthiazolyl, Pyridazinyl.
Als Heteroarylalkyl seien genannt Heteroarylmethyl, Heteroarylethyl mit bis zu 6 Ringatomen und N, O, S, insbesondere N als Heteroatomen, insbesondere gegebenenfalls substituiertes Heteroaryl wie unter Heteroaryl definiert.
Als Substituenten seien beispielhaft und vorzugsweise aufgeführt:

Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor oder Brom, insbesondere Fluor stehen, wie Trifluormethyl, Hydroxy;

Halogen, vorzugsweise Fluor, Chlor, Brom und Iod, insbesondere Fluor, Chlor und Brom, Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methylethylamino, n- und i-Propylamino und Methyl-n-butylamino; Carboxyl; Carbalkoxy mit vorzugsweise 2 bis 4, insbesondere 2 oder 3 Kohlenstoffatomen, wie Carbomethoxy und Carboethoxy;

Sulfo (-$SO_3H$); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl sowie Heteroarylamino und Heteroarylalkylamino wie Chlorpyridylamino und Chlorpyridylmethylamino.

A steht für Wasserstoff oder für einen gegebenenfalls substituierten Rest aus der Reihe Acyl, Alkyl, Aryl, die bevorzugt die bei R angegebenen Bedeutungen haben, A steht ferner für eine bifunktionelle Gruppe. Genannt sei gegebenenfalls substituiertes Alkylen mit 1 bis 4, insbesondere 1 bis 2 C-Atomen, wobei als Substituenten die weiter oben aufgezählten Substituenten genannt seien (und wobei die Alkylengruppen durch Heteroatome aus der Reihe N, O, S unterbrochen sein können).

A und Z können gemeinsam mit den Atomen, an welche sie gebunden sind, einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei Alkyl der N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.

Als Beispiele für die Verbindungen der Formel (II), in denen A und Z gemeinsam mit den Atomen, an die sie gebunden sind einen Ring bilden, seien die folgenden genannt:

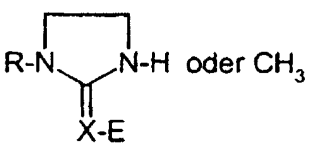

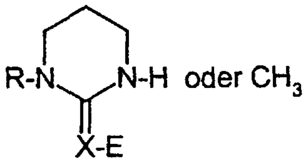

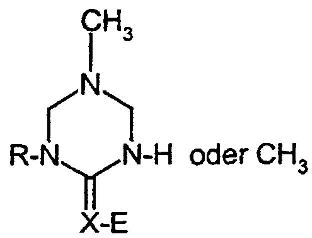

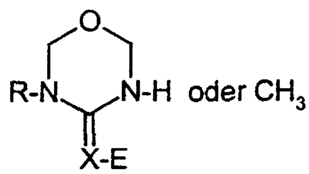

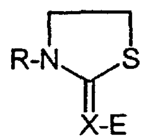

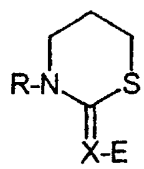

in welchen

E, R und X die oben und weiter unten genannten Bedeutungen haben.

E steht für einen elektronenziehenden Rest, wobei insbesondere $NO_2$, CN, Halogenalkylcarbonyl wie Halogen-$C_1$-$C_4$-alkylcarbonyl, beispielsweise $COCF_3$, Alkylsulfonyl (z.B. $SO_2$-$CH_3$), Halogenalkylsulfonyl (z.B. $SO_2CF_3$) und ganz besonders $NO_2$ oder CN genannt seien.

X steht für -CH= oder -N= .

Z steht für einen gegebenenfalls substituierten Rest aus der Reihe Alkyl, -OR, -SR, -NRR, wobei R und die Substituenten bevorzugt die oben angegebene Bedeutung haben.

Z kann außer dem obengenannten Ring gemeinsam mit dem Atom, an welches es gebunden ist und dem Rest

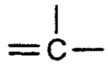

an der Stelle von X einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei die Alkyl oder N-Alkyl-Gruppe vorzugsweise 1 bis 4, vorzugsweise 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder. Als Beispiele für den heterocyclischen Ring seien Pyrrolidin, Piperidin, Piperazin, Hexamethylenimin, Morpholin und N-Methylpiperazin genannt.

Besonders bevorzugt handelt es sich bei den Agonisten und Antagonisten der nicotinergen Acetylcholinrezeptoren um Verbindungen der Formel (II), worin

R für

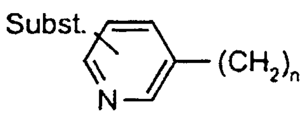 oder 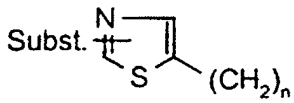 oder 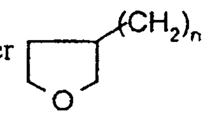

steht, wobei

n für 0, 1 oder 2, bevorzugt für 1 steht,

Subst. für einen der oben aufgeführten Substituenten, besonders für Halogen, insbesondere für Chlor steht und A, Z, X und E die oben angegebene Bedeutung haben.

R steht insbesondere für

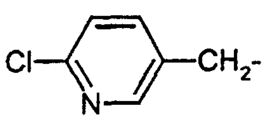 oder 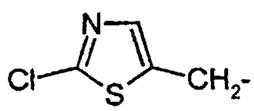

oder

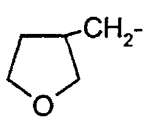.

Im einzelnen seien folgende Verbindungen genannt:

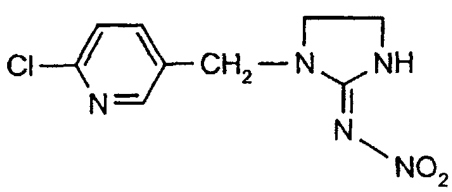

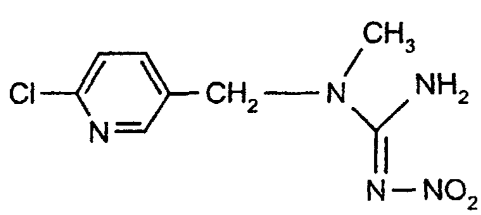

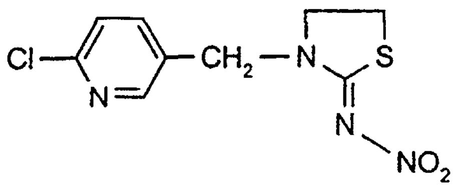

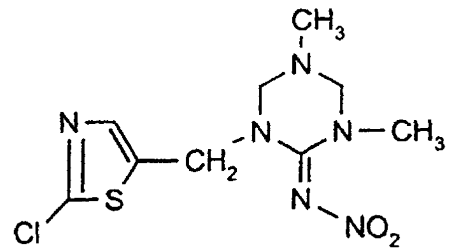

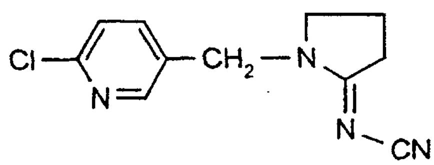

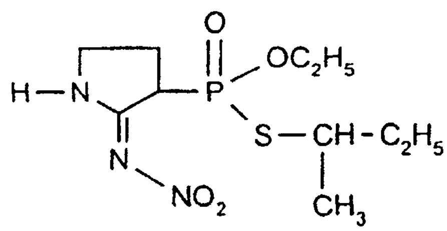

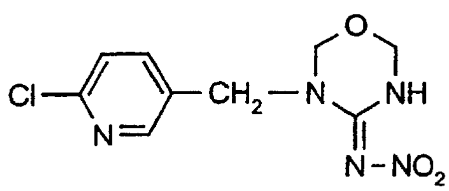

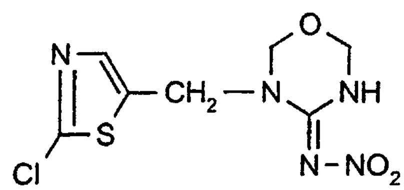

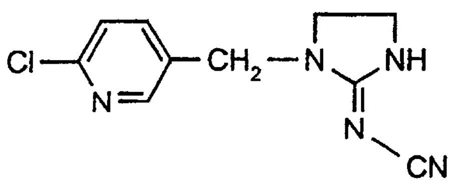

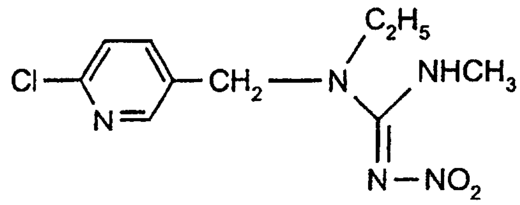

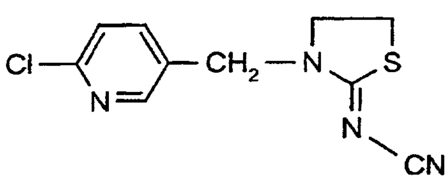

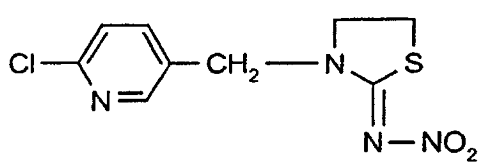

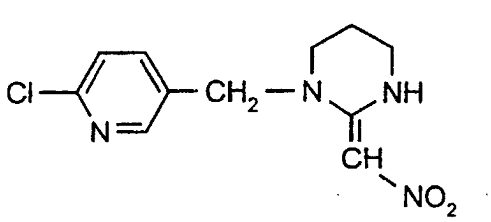

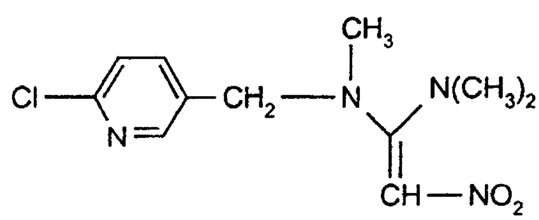

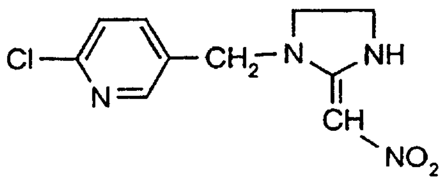

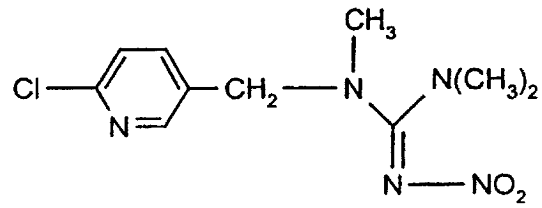

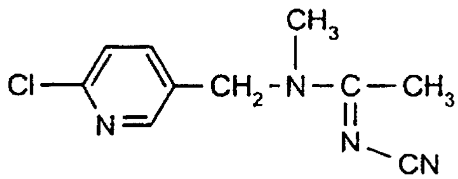

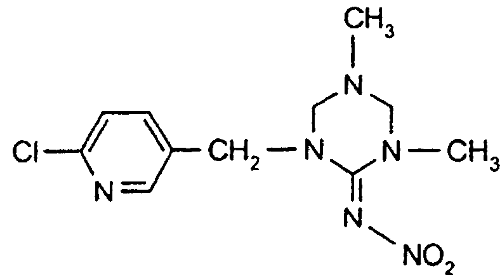

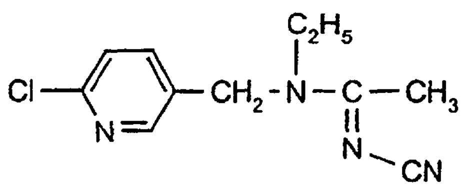

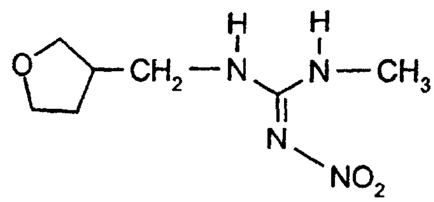

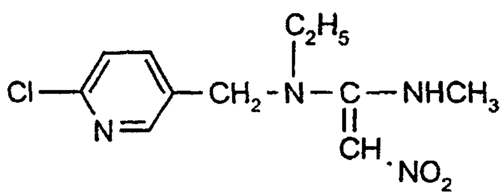

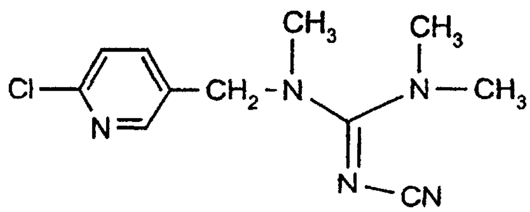

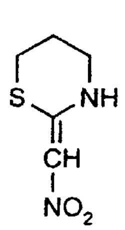

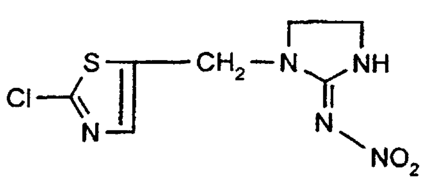

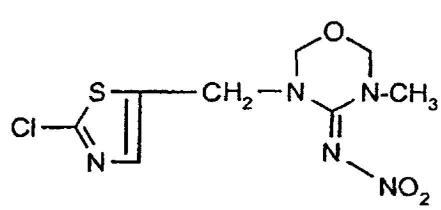

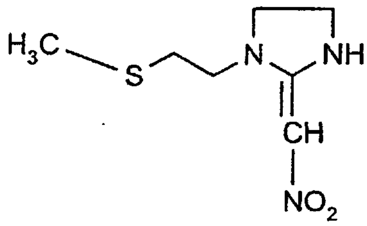

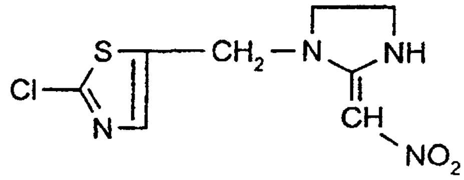

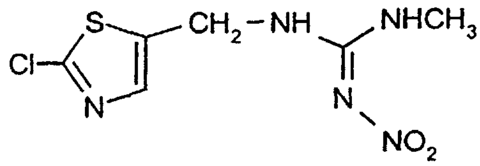

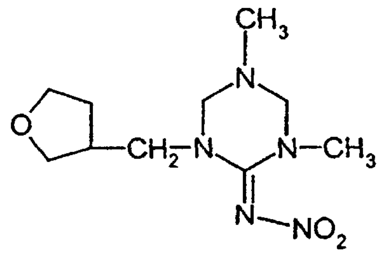

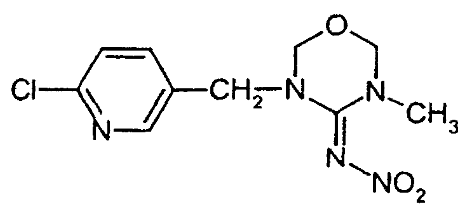

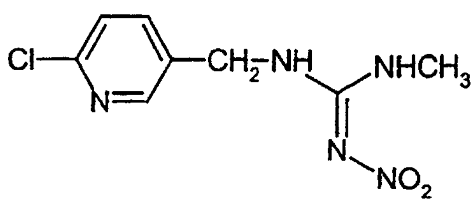

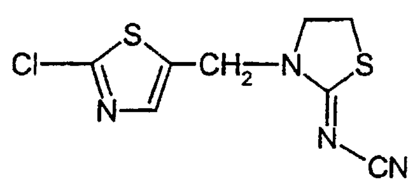

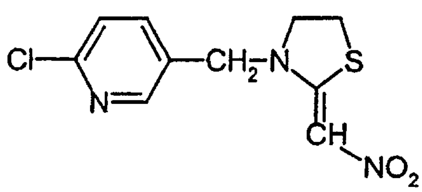

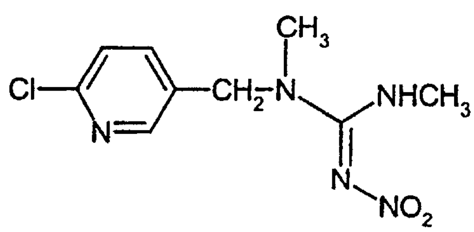

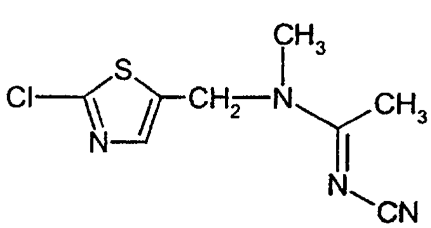

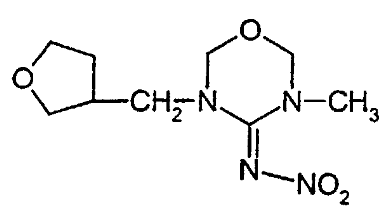

Ganz besonders bevorzugte Agonisten und Antagonisten der nicotinergen Acetylcholinrezeptoren sind Verbindungen der folgenden Formeln:

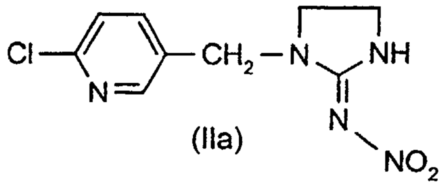

(IIa)

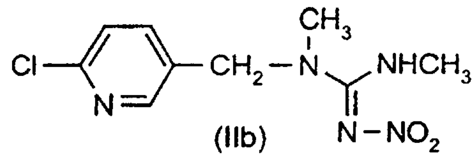

(IIb)

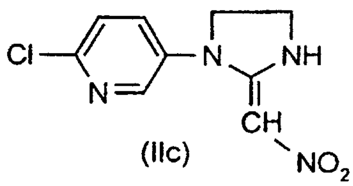

(IIc)

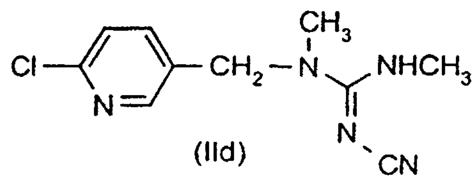

(IId)

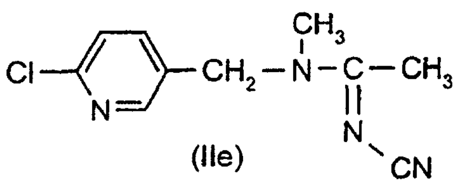

(IIe)

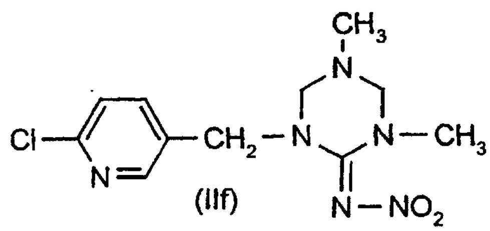

(IIf)

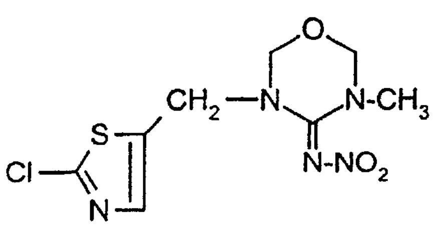

(IIg)

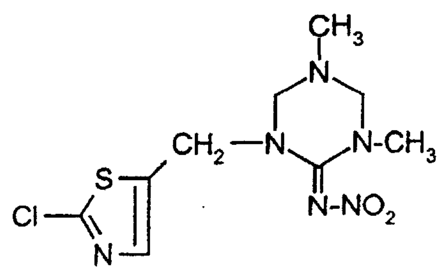

(IIh)

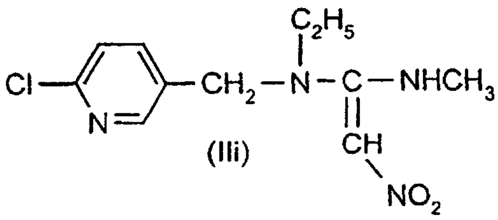

(IIi)

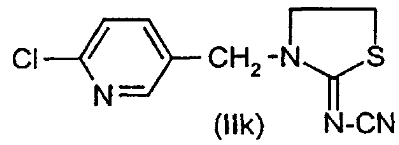

(IIk)

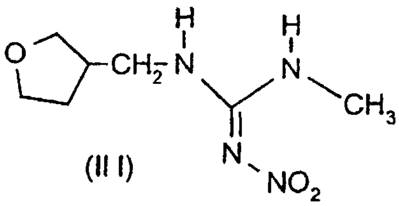

(II l)

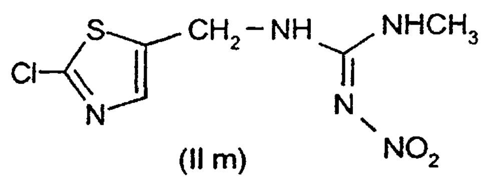

(II m)

insbesondere eine Verbindung der folgenden Formeln

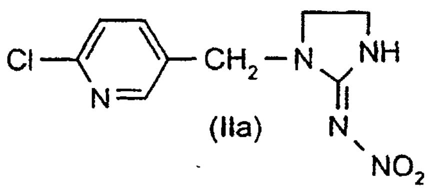

(IIa)

oder

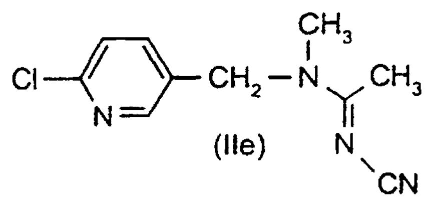

(IIe)

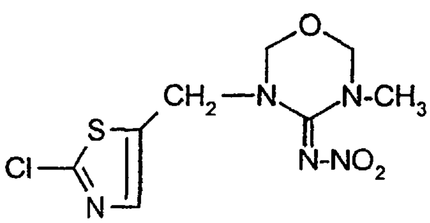

(IIg)

oder

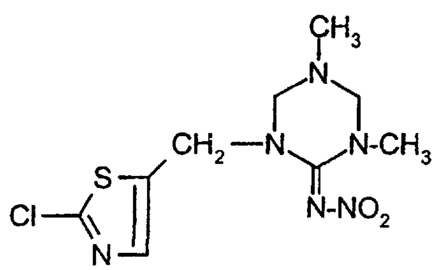

(IIh)

oder

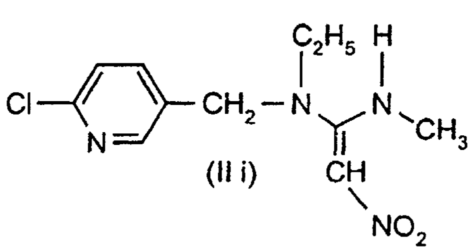

(II i)

oder

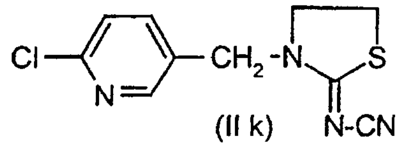

(II k)

oder

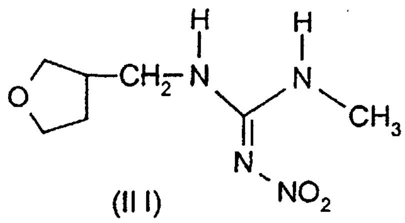

(II l)

oder

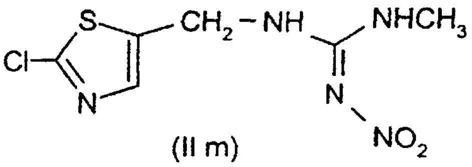

(II m)

Ganz besonders bevorzugt sind die Verbindungen der Formeln (IIa), (IIk), (IIm).

Weiterhin ganz besonders bevorzugt sind die Verbindungen der Formeln (IIe), (IIg). (IIh), (III), (IIc).

Eine besonders bevorzugte Mischung enthält die Verbindung der Formel (Ia) und die Verbindung der Formel (IIa);

eine weiter besonders bevorzugte Mischung enthält die Verbindung der Formel (Ia) und die Verbindung der Formel (IIk); weiter besonders bevorzugt sind Mischungen enthaltend die Verbindung der Formel (Ia) und die Verbindung der Formel (IIm).

Die Wirkstoffinischungen eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorratsschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp.
Aus der Ordnung der Anoplura z.B. Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Phylloxera vastatrix, Pemphigus spp., Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofinannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermesles spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Das Verhältnis der eingesetzten Verbindungen der Formel (I) und den Verbindungen der Formel (II), sowie die Gesamtmenge der Mischung ist von der Art und dem Vorkommen der Schädlinge abhängig. Die optimalen Verhältnisse und Gesamteinsatzmengen können bei jeder Anwendung jeweils durch Testreihen ermittelt werden. Im allgemeinen ist das Verhältnis der Verbindungen der Formel (I) und den Verbindungen der Formel (II) 1:100 bis 100:1, vorzugsweise 1:25 bis 25:1 und besonders bevorzugt 1:5 bis 5:1. Hierbei handelt es sich um Gewichtsteile.

Die erfindungsgemäßen Wirkstoffmischungen können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe. Im einzelnen seien die weiter obengenannten Insektizide und Fungizide als Zumischpartner genannt.

Als Insektizide, die gegebenenfalls zugemischt werden können handelt es sich beispielsweise um:

Phosphorsäureester wie Azinphos-ethyl, Azinphos-methyl, a-1(4-Chlorphenyl)-4-(O-ethyl, S-propyl)phosphoryloxy-pyrazol, Chlorpyrifos, Coumaphos, Demeton, Demeton-S-methyl, Diazinon, Dichlorvos, Dimethoate, Ethoate, Ethoprophos, Etrimfos, Fenitrothion, Fenthion, Heptenophas, Parathion, Parathion-methyl, Phosalone, Phoxim, Pirimiphos-ethyl, Pirimiphos-methyl, Profenofos, Prothiofos, Sulfprofos, Triazophos und Trichlorphon;

Carbamate wie Aldicarb, Bendiocarb, a-2-(1-Methylpropyl)-phenylmethylcarbamat, Butocarboxim, Butoxycarboxim, Carbaryl, Carbofuran, Carbosulfan, Cloethocarb, Isoprocarb, Methomyl, Oxamyl, Pirimicarb, Promecarb, Propoxur und Thiodicarb;

Organosiliciumverbindungen, vorzugsweise Dimethyl(phenyl)silyl-methyl-3-phenoxybenzylether wie Dimethyl-(4-ethoxyphenyl)-silylmethyl-3-phenoxybenzylether oder
(Dimethylphenyl)-silyl-methyl-2-phenoxy-6-pyridylmethylether wie z.B. Dimethyl-(9-ethoxy-phenyl)-silylmethyl-2-phenoxy-6-pyridylmethylether oder [(Phenyl)-3-(3-phenoxyphenyl)-propyl](dimethyl)-silane wie z.B. (4-Ethoxyphenyl)-[3-(4-fluoro-3-phenoxyphenyl-propyl]dimethyl-silan, Silafluofen;
Pyrethroide wie Allethrin, Alphamethrin, Bioresmethrin, Byfenthrin, Cycloprothrin, Cyfluthrin, Decamethrin, Cyhalothrin, Cypermethrin, Deltamethrin, Alpha-cyano-3-phenyl-2-methylbenzyl-2,2-dimethyl-3-(2-chlor-2-trifluor-methylvinyl)cyclopropan-Carboxylat, Fenpropathrin, Fenfluthrin, Fenvalerate, Flucythrinate, Flumethrin, Fluvalinate, Permethrin, Resmethrin und Tralomethrin;
Nitroimine und Nitromethylene wie 1-[(6-Chlor-3-pyridinyl)-methyl]-4,5-dihydro-N-nitro-1H-imidazol-2-amin (Imidacloprid), N-[(6-Chlor-3-pyridyl)methyl-]$N^2$-cyano-$N^1$-methylacetamide (NI-25);
Abamectin, AC 303.630, Acephate, Acrinathrin, Alanycarb, Aldoxycarb, Aldrin, Amitraz, Azamethiphos, Bacillus thuringiensis, Phosmet, Phosphamidon, Phosphine, Prallethrin, Propaphos, Propetamphos, Prothoate, Pyraclofos, Pyrethrins, Pyridaben, Pyridafenthion, Pyriproxyfen, Quinalphos, RH-7988, Rotenone, Sodium fluoride, Sodium hexafluorosilicate, Sulfotep, Sulfuryl fluoride, Tar Oils, Teflubenzuron, Tefluthrin, Temephos, Terbufos, Tetrachlorvinphos, Tetramethrin, O-2-tert.-Butylpyrimidin-5-yl-o-isopropyl-phosphorothiate, Thiocyclam, Thiofanox, Thiometon, Tralomethrin, Triflumuron, Trimethacarb, Vamidothion, Verticillium Lacanii, XMC, Xylylcarb, Benfuracarb, Bensultap, Bifenthrin, Bioallethrin, MERbioallethrin (S)-cyclopentenyl isomer, Bromophos, Bromophos-ethyl, Buprofezin, Cadusafos, Calcium Polysulfide, Carbophenothion, Cartap, Chinomethionat, Chlordane, Chlorfenvinphos, Chlorfluazuron, Chlormephos, Chloropicrin, Chlorpyrifos, Cyanophos, Beta-Cyfluthrin, Alpha-cypermethrin, Cyophenothrin, Cyromazine, Dazomet, DDT, Demeton-S-methylsulphon, Diafenthiuron, Dialifos, Dicrotophos, Diflubenzuron, Dinoseb, Deoxabenzofos, Diaxacarb, Disulfoton, DNOC, Empenthrin, Endosulfan, EPN, Esfenvalerate, Ethiofencarb, Ethion, Etofenprox, Fenobucarb, Fenoxycarb, Fensulfothion, Spinosynen, Flucycloxuron, Flufenprox, Flufenoxuron, Fonofos, Formetanate, Formothion, Fosmethilan, Furathiocarb, Heptachlor, Hexaflumuron, Hydramethylnon, Hydrogen Cyanide, Hydroprene, IPSP, Isazofos, Isofenphos, Isoprothiolane, Isoxathion, Iodfenphos, Kadethrin, Lindane, Malathion, Mecarbam, Mephosfolan, Mercurous, chloride, Metam, Metarthizium, anisopliae, Methacrifos, Methamidophos, Methidathion, Methiocarb, Methoprene, Methoxychlor, Methyl isothiocyanate, Metholcarb, Mevinphos, Monocrotophos, Naled, Neodiprion sertifer NPV, Nicotine, Omethoate, Oxydemeton-methyl, Pentachlorophenol, Petroleum oils, Phenothrin, Phenthoate, Phorate.

Dabei können die gegebenenfalls noch zumischbaren weiteren Insektizide auch aus der Klasse der Verbindungen der allgemeinen Formel (I) stammen.
Als gegebenenfalls noch zumischbaren Fungizide kommen vorzugsweise in Frage:

**Triazole wie:**

Azaconazole, Propiconazole, Tebuconazole, Cyproconazole, Metconazole, Amitrole, Azocyclotin, BAS 480F, Bitertanol, Difenoconazole, Fenbuconazole, Fenchlorazole, Fenethanil, Fluquinconazole, Flusilazole, Flutriafol, Imibenconazole, Isozofos, Myclobutanil, Paclobutrazol, (±)-cis-1-(4-chlorphenyl)-2-(1H-1,2,4-triazol-1-yl)-cycloheptanol, Tetraconazole, Triadimefon, Triadimenol, Triapenthenol, Triflumizole, Triticonazole, Uniconazole sowie deren Metallsalze und Säureaddukte.

**Imidazole wie:**

Imazalil, Pefurazoate, Prochloraz, Triflumizole, 2-(1-tert-Butyl)-1-(2-chlorphenyl)-3-(1,2,4-triazol-1-yl)-propan-2-ol, Thiazolcarboxanilide wie 2',6'-Dibromo-2-methyl-4-trifluoromethoxy-4'-trifluoromethyl-1,3-thiazole-5-carboxanilide, 1-Imidazolyl-1-(4'chlorophenoxy)-3,3-dimethylbutan-2-on sowie deren Metallsalze und Säureaddukte.

Methyl(E)-2-[2-[6-(2-cyanophenoxy)pyrimidin-4-yloxy]phenyl]3-methoxyacrylate, methyl(E)-2-[2-[6-(2-thioamidophenoxy)pyrimidin-4-yloxy]phenyl]-3-methoxyacrylate, methyl(E)-2-[2-[6-(2-fluorophenoxy)pyrimidin-4-yloxy]phenyl]-3-methoxyacrylate, methyl(E)-2-[2-[6-(2,6-difluorophenoxy)pyrimidin-4-yloxy]phenyl]-3-methoxyacrylate, methyl(E)-2-[2-[3-(pyrimidin-2-yloxy)phenoxy]phenyl]-3-methoxyacrylate, methyl(E)-2-[2-[3-(5-methylpyrimidin-2-yloxy)-phenoxy]phenyl]-3-methoxyacrylate, methyl(E)-2-[2-[3-(phenyl-sulfonyloxy)phenoxy]phenyl]-3-methoxyacrylate, methyl(E)-2-[2-[3-(4-nitrophenoxy)phenoxy]phenyl]-3-methoxyacrylate, methyl(E)-2-[2-phenoxyphenyl]-3-methoxyacrylate, methyl(E)-2-[2-(3,5-dimethylbenzoyl)pyrrol-1-yl]-3-methoxyacrylate, methyl(E)-2-[2-(3-methoxyphenoxy)phenyl]-3-methoxyacrylate, methyl(E)-2-[2-(2-pheny]ethen-1-yl)-phenyl]-3-methoxyacrylate, methyl(E)-2-[2-(3,5-dichlorophenoxy)pyridin-3-yl]-3-methoxyacrylate, methyl(E)-2-(2-(3-(1,1,2,2-tetrafluoroethoxy)phenoxy)phenyl)-3-methoxyacrylate, methyl(E)-2-(2-[3-(alpha-hydroxybenzyl)phenoxy]phenyl)-3-methoxyacrylate, methyl(E)-2-(2-(4-phenoxypyridin-2-yloxy)phenyl)-3-methoxyacrylate, methyl(E)-2-[2-(3-n-propyloxyphenoxy)phenyl]3-methoxyacrylate, methyl(E)-2-[2-(3-isopropyloxyphenoxy)phenyl]-3-methoxyacrylate, methyl(E)-2-[2-[3-(2-fluorophenoxy)pehnoxy]phenyl]-3-methoxyacrylate, methyl(E)-2-[2-(3-ethoxyphenoxy)-phenyl]-3-methoxyacrylate,methyl(E)-2-[2-(4-tert.-butylpyridin-2-yloxy)phenyl]-3-methoxyacrylate,methyl(E)-2-[2-[3-(3-cyanophenoxy)phenoxy]phenyl]-3-methoxyacrylate, methyl(E)-2-[2-(3-methylpyridin-2-yloxymethyl)phenyl]-3-methoxyacrylate, methyl(E)-2-[2-[6-(2-methylphenoxy)pyrimidin-4-yloxy]phenyl]-3-methoxyacrylate, methyl(E)-2-[2-(5-bromopyridin-2-yloxymethyl)phenyl]-3-methoxyacrylate, methyl(E)-2-(2-(3-(3-iodopyridin-2-yloxy)phenoxy)phenyl]-3-methoxyacrylate, methyl(E)-2-[2-[6-(2-chloropyridin-3-yloxy)pyrimidin-4-yloxy]phenyl]-3-methoxyacrylate, (E),(E)methyl-2-[2-(5,6-dimethylpyrazin-2-ylmethyloximinomethyl)phenyl]-3-methoxyacrylate, (E)-methyl-2-{2-[6-(6-methylpyridin-2-yloxy)pyrimidin-4-yloxy]phenyl}-3-methoxyacrylate, (E),(E)methyl-2-{2-(3-methoxyphenyl)methyloximinomethyl]phenyl}-3-methoxyacrylate, (E)methyl-2-{2-(6-(2-azidophenoxy)-pyrimidin-4-yloxy]phenyl}3-methoxyacrylate, (E),(E)methyl-2-{2-[6-phenylpyrimidin-4-yl)-methyloximinomethyl]phenyl}-3-methoxyacrylate, (E),(E)methyl-2-{2-[(4-chlorophenyl)-methyloximinomethyl]phenyl}-3-methoxyacrylate, (E)methyl-2-{2-[6-(2-n-propylphenoxy)-1,3,5-triazin-4-yloxy]phenyl}-3-methoxyacrylate, (E),(E)-methyl-2- {2-[(3-nitrophenyl)methyloximinomethyl]phenyl }-3-methoxyacrylate;

**Succinat-Dehydrogenase Inhibitoren wie:**

Fenfuram, Furcarbanil, Cyclafluramid, Furmecyclox, Seedvax, Metsulfovax, Pyrocarbolid, Oxycarboxin, Shirlan, Mebenil (Mepronil), Benodanil, Flutolanil (Moncut);

Naphthalin-Derivate wie Terbinafine, Naftifine, Butenafine, 3-Chloro-7-(2-aza-2,7,7-trimethyl-oct-3-en-5-in);

Sulfenamide wie Dichlofluanid, Tolylfluanid, Folpet, Fluorfolpet; Captan, Captofol;

Benzimidazole wie Carbendazim, Benomyl, Furathiocarb, Fuberidazole, Thiophonatmethyl, Thiabendazole oder deren Salze;

Morpholinderivate wie Fenpropimorph, Falimorph, Dimethomorph, Dodemorph, Aldimorph, Fenpropidin und ihre arylsulfonsauren Salze, wie z.B. p-Toluolsulfonsäure und p-Dodecylphenyl-sulfonsäure;

Dithiocarbamate, Cufraneb, Ferbam, Mancopper, Mancozeb, Maneb, Metam, Metiram, Thiram Zeneb, Ziram;

Benzthiazole wie 2-Mercaptobenzothiazol;

Benzamide wie 2,6-Dichloro-N-(4-trifluoromethylbenzyl)-benzamide;

Borverbindungen wie Borsäure, Borsäureester, Borax;

Formaldehyd und Formaldehydabspaltende Verbindungen wie Benzylalkoholmono-(poly)-hemiformal, Oxazolidine, Hexa-hydro-S-triazine, N-Methylolchloracetamid, Paraformadehyd, Nitropyrin, Oxolinsäure, Tecloftalam;

Tris-N-(cyclohexyldiazeniumdioxy)-aluminium, N-(Cyclo-hexyldiazeniumdioxy)-tributylzinn bzw. K-Salze, Bis-N-(cyclohexyldiazeniumdioxy)-kupfer;

N-Methylisothiazolin-3-on, 5-Chlor-N-methylisothiazolin-3-on, 4,5-Dichloro-N-octylisothiazolin-3-on, N-Octylisothiazolin-3-on, 4,5-Trimethylen-isothiazolinone, 4,5-Benzisothiazolinone, N-Methylolchloracetamid;

Aldehyde wie Zimtaldehyd, Formaldehyd, Glutardialdehyd, β-Bromzimtaldehyd; Thiocyanate wie Thiocyanatomethylthiobenzothiazol, Methylenbisthiocyanat, usw;

quartäre Ammoniumverbindungen wie Benzyldimethyltetradecylammoniumchlorid, Benzyldimethyldodecylammoniumchlorid, Didecyldimethaylammoniumchlorid;

Iodderivate wie Diiodmethyl-p-tolylsulfon, 3-Iod-2-propinyl-alkohol, 4-Chlorphenyl-3-iodpropargylformal, 3-Brom-2,3-diiod-2-propenylethylcarbamat, 2,3,3-Triiodallylalkohol, 3-Brom-2,3-diiod-2-propenylalkohol, 3-Iod-2-propinyl-n-butylcarbamat, 3-Iod-2-propinyl-n-hexylcarbamat, 3-Iod-2-propinyl-cyclohexylcarbamat, 3-Iod-2-propinyl-phenylcarbamat;

Phenolderivate wie Tribromphenol, Tetrachlorphenol, 3-Methyl-4-chlorphenol, 3,5-Dimethyl-4-chlorphenol, Phenoxyethanol, Dichlorphen, o-Phenylphenol, m-Phenylphenol, p-Phenylphenol, 2-Benzyl-4-chlorphenol und deren Alkali- und Erdalkalimetallsalze;

Mikrobizide mit aktivierter Halogengruppe wie Chloracetamid, Bronopol, Bronidox, Tectamer wie 2-Brom-2-nitro-1,3-propandiol, 2-Brom-4'-hydroxy-acetophenon, 2,2-Dibrom-3-nitril-propionamid, 1,2-Dibrom-2,4-dicyanobutan, β-Brom-β-nitrostyrol;

Pyridine wie 1-Hydroxy-2-pyridinthion (und ihre Na-, Fe-, Mn-, Zn-Salze), Tetrachlor-4-methylsulfonylpyridin, Pyrimethanol, Mepanipyrim, Dipyrithion, 1-Hydroxy-4-methyl-6-(2,4,4-trimethylpentyl)-2(1H)-pyridin;

Metallseifen wie Zinn-, Kupfer-, Zinknaphtenat, -octoat, 2-ethylhexanoat, -oleat, -phosphat, -benzoat;

Metallsalze wie Kupferhydroxycarbonat, Natriumdichromat, Kaliumdichromat, Kaliumchromat, Kupfersulfat, Kupferchlorid, Kupferborat, Zinkfluorosilikat, Kupferfluorosilikat, insbesondere Mischung mit Fixiermitteln;

Oxide wie Tributylzinnoxid, $Cu_2O$, CuO, ZnO;

Dialkyldithiocarbamate wie Na- und Zn-Salze von Dialkyldithiocarbamaten, Tetramethylthiuramdisulfid, Kalium-N-methyl-dithiocarbamat;
Nitrile wie 2,4,5,6-Tetrachlorisophthalodinitril, Dinatrium-cyano-dithioimidocarbamat;
Chinoline wie 8-Hydroxychinolin und deren Cu-Salze;
Mucochlorsäure, 5-Hydroxy-2(5H)-furanon;
4,5-Dichlorodithiazolinon, 4,5-Benzdithiazolinon, 4,5-Trimethylendithiazolinon, 4,5-Dichlor-(3H)-1,2-dithiol-3-on, 3,5-Dimethyl-tetrahydro-1,3,5-thiadiazin-2-thion,
N-(2-p-Chlorbenzoylethyl)-hexaminiumchlorid, Kalium-N-hydroxymethyl-N'-methyl-dithiocarbamat,
2-Oxo-2-(4-hydroxy-phenyl)acethydroximsäure-chlorid,
Phenyl-(2-chlor-cyan-vinyl)sulfon,
Phenyl-(1,2-dichlor-2-cyan-vinyl)sulfon;
Ag, Zn oder Cu-haltige Zeolithe allein oder eingeschlossen in polymere Wirkstoffe, oder auch Mischungen aus mehrern der oben genannten Fungizide.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Wirkstoffinischungen können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kaltund Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoffinischung, vorzugsweise zwischen 0,5 und 90 Gewichtsprozent Wirkstoffmischung.

Die erfindungsgemäßen Mischungen können über das Blatt angewendet werden.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z. B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Beim Einsatz der erfindungsgemäßen Wirkstoffkombinationen können die Aufwandmengen je nach Applikationsart innerhalb eines größeren Bereichs variiert werden. Bei der Behandlung von Pflanzenteilen liegen die Aufwandmengen an Wirkstoffkombination im allgemeinen zwischen 0,1 und 10 000 g/ha, vorzugsweise zwischen 10 und 1 000 g/ha.

Die gute insektizide und akarizide Wirkung der erfindungsgemäßen Wirkstoffkombinationen geht aus den nachfolgenden Beispielen hervor. Während die einzelnen Wirkstoffe in der Wirkung Schwächen aufweisen, zeigen die Kombinationen eine Wirkung, die über eine einfache Wirkungssummierung hinausgeht.

Ein synergistischer Effekt liegt bei Insektiziden und Akariziden immer dann vor, wenn die Wirkung der Wirkstoffkombinationen größer ist als die Summe der Wirkungen der einzeln applizierten Wirkstoffe.

Die zu erwartende Wirkung für eine gegebene Kombination zweier Wirkstoffe kann nach S.R. Colby, Weeds 15 (1967), 20-22) wie folgt berechnet werden:
Wenn

- X den Wirkungsgrad beim Einsatz des Wirkstoffes A in einer Aufwandmenge von m g/ha oder in einer Konzentration von m ppm bedeutet,
- Y den Wirkungsgrad beim Einsatz des Wirkstoffes B in einer Aufwandmenge von n g/ha oder in einer Konzentration von n ppm bedeutet und
- E den Wirkungsgrad beim Einsatz der Wirkstoffe A und B in Aufwandmengen von m und n g/ha oder in einer Konzentration von m und n ppm bedeutet,

dann ist

$$E = X + Y - \frac{X \cdot Y}{100}$$

Dabei wird der Wirkungsgrad in % ermittelt. Es bedeutet 0 % ein Wirkungsgrad, der demjenigen der Kontrolle entspricht, während ein Wirkungsgrad von 100 % bedeutet, dass kein Befall beobachtet wird.

Ist die tatsächliche Wirkung größer als berechnet, so ist die Kombination in ihrer Wirkung überadditiv, d.h. es liegt ein synergistischer Effekt vor. In diesem Fall muss der tatsächlich beobachtete Wirkungsgrad größer sein als der aus der oben angeführten Formel errechnete Wert für den erwarteten Wirkungsgrad (E).

## Beispiel A

| **Aphis gossypii-Test** | |
|---|---|
| Lösungsmittel | 3 Gewichtsteile Dimethylformamid |
| Emulgator | 1 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschten Konzentrationen.

Baumwollblätter (Gossypium hirsutum), die stark von der Baumwollblattlaus (Aphis gossypii) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden. Die ermittelten Abtötungswerte verrechnet man nach der Colby-Formel.

Bei diesem Test zeigt z.B. die folgende Wirkstoffkombination gemäß vorliegender Anmeldung eine synergistisch verstärkte Wirksamkeit im Vergleich zu den einzeln angewendeten Wirkstoffen:

Tabelle A

| Blatt 1<br>pflanzenschädigende Insekten<br>**Aphis gossypii-Test** | | |
|---|---|---|
| Wirkstoffe | Wirkstoffkonzentration in ppm | Abtötungsgrad in % nach 6$^d$ |
| Bsp. (Ia) bekannt | 1,6 | 0 |
| Bsp. (IIa) bekannt | 1,6 | 85 |
| Bsp. (Ia) + Bsp. (IIa) erfindungsgemäß | 1,6 + 1,6 | gef.* 95 ber.** 85 |
| gef.* = gefundene Wirkung<br>ber.** = nach der Colby-Formel berechnete Wirkung | | |

Tabelle A

| Blatt 2<br>pflanzenschädigende Insekten<br>**Aphis gossypii-Test** | | |
|---|---|---|
| Wirkstoffe | Wirkstoffkonzentration in ppm | Abtötungsgrad in % nach 6$^d$ |
| Bsp. (Ia) bekannt | 1,6 | 0 |
| Bsp. (IIk) bekannt | 1,6 | 55 |
| Bsp. (Ia) + Bsp. (IIk) erfindungsgemäß | 1,6 + 1,6 | gef.* 95 ber.** 55 |
| gef.* = gefundene Wirkung<br>ber.** = nach der Colby-Formel berechnete Wirkung | | |

**Beispiel B**

| **Aphis gossypii-Test/Larvalmortalität** | |
|---|---|
| Lösungsmittel: | 3 Gewichtsteile Dimethylformamid |
| Emulgator: | 1 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschten Konzentrationen.

Baumwollblätter (Gossypium hirsutum), die stark von Adulten und Larven der Baumwollblattlaus (Aphis gossypii) befallen sind, werden durch Tauchen in die Wirkstoffzubereitung der gewünschten Konzentration behandelt.

Nach der gewünschten Zeit wird die Abtötung der Larven in % bestimmt. Dabei bedeutet 100 %, dass alle Larven abgetötet wurden; 0 % bedeutet, daß keine Larven abgetötet wurden. Die ermittelten Abtötungswerte verrechnet man nach der Colby-Formel.

Bei diesem Test zeigt z.B. die folgende Wirkstoffkombination gemäß vorliegender Anmeldung eine synergistisch verstärkte Wirksamkeit im Vergleich zu den einzeln angewendeten Wirkstoffen:

Tabelle B

| Blatt 1<br>pflanzenschädigende Insekten<br>**Aphis gossypii-Test/Larvalmortalität** | | |
|---|---|---|
| Wirkstoffe | Wirkstoffkonzentration in ppm | Abtötungsgrad in % nach 6$^d$ |
| Bsp. (Ia) bekannt | 1,6 | 0 |
| Bsp. (IIa) bekannt | 1,6 | 80 |

Tabelle B (fortgesetzt)

| Blatt 1<br>pflanzenschädigende Insekten<br>**Aphis gossypii-Test/Larvalmortalität** | | |
|---|---|---|
| Wirkstoffe | Wirkstoffkonzentration in ppm | Abtötungsgrad in % nach 6$^d$ |
| Bsp. (Ia) + Bsp. (IIa) erfindungsgemäß | 1,6 + 1,6 | gef.* 95 ber.** 80 |
| gef.* = gefundene Wirkung<br>ber.** = nach der Colby-Formel berechnete Wirkung | | |

Tabelle B

| Blatt 2<br>pflanzenschädigende Insekten<br>**Aphis gossypii-Test/Larvalmortalität** | | |
|---|---|---|
| Wirkstoffe | Wirkstoffkonzentration in ppm | Abtötungsgrad in % nach 6$^d$ |
| Bsp. (Ia) bekannt | 1,6 | 0 |
| Bsp. (IIk) bekannt | 1,6 | 60 |
| Bsp. (Ia) + Bsp. (IIk) erfindungsgemäß | 1,6 + 1,6 | gef.* 95 ber.** 60 |
| gef.* = gefundene Wirkung<br>ber.** = nach der Colby-Formel berechnete Wirkung | | |

**Beispiel C**

| **Myzus-Test** | |
|---|---|
| Lösungsmittel | 3 Gewichtsteile Dimethylformamid |
| Emulgator | 1 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschten Konzentrationen.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Tiere abgetötet wurden; 0 % bedeutet, daß keine Tiere abgetötet wurden. Die ermittelten Abtötungswerte verrechnet man nach der Colby-Formel.

Bei diesem Test zeigt z.B. die folgende Wirkstoffkombination gemäß vorliegender Anmeldung eine synergistisch verstärkte Wirksamkeit im Vergleich zu den einzeln angewendeten Wirkstoffen:

Tabelle C

| pflanzenschädigende Insekten<br>**Myzus-Test** | | |
|---|---|---|
| Wirkstoffe | Wirkstoffkonzentration in ppm | Abtötungsgrad in % nach 1$^d$ |
| Bsp. (Ia) bekannt | 1,6 | 0 |
| Bsp. (IIa) bekannt | 1,6 | 70 |
| Bsp. (Ia) + Bsp. (IIa) erfindungsgemäß | 1,6 + 1,6 | gef.* 98 ber.** 70 |
| gef.* = gefundene Wirkung<br>ber.** = nach der Colby-Formel berechnete Wirkung | | |

**Beispiel D**

| **Myzus-Test/Larvalmortalität** | |
|---|---|
| Lösungsmittel | 3 Gewichtsteile Dimethylformamid |
| Emulgator | 1 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschten Konzentrationen.

Kohlblätter (Brassica oleracea), die stark von Adulten und Larven der Pfirsichblattlaus (Myzus persicae) befallen sind, werden durch Tauchen in die Wirkstoffzubereitung der gewünschten Konzentration behandelt

Nach der gewünschten Zeit wird die Abtötung der Larven in % bestimmt. Dabei bedeutet 100 %, dass alle Larven abgetötet wurden; 0 % bedeutet, dass keine Larven abgetötet wurden. Die ermittelten Abtötungswerte verrechnet man nach der Colby-Formel.

Bei diesem Test zeigt z.B. die folgende Wirkstoffkombination gemäß vorliegender Anmeldung eine synergistisch verstärkte Wirksamkeit im Vergleich zu den einzeln angewendeten Wirkstoffen:

Tabelle D

| pflanzenschädigende Insekten<br>**Myzus-Test/Larvalmortalität** | | |
|---|---|---|
| Wirkstoffe | Wirkstoffkonzentration in ppm | Abtötungsgrad in % nach 6[d] |
| Bsp. (Ia) bekannt | 0,32 | 0 |
| Bsp. (IIa) bekannt | 0,32 | 0 |
| Bsp. (Ia) + Bsp. (IIa) erfindungsgemäß | 0,32 + 0,32 | gef.* 55 ber.** 0 |
| gef.* = gefundene Wirkung<br>ber.** = nach der Colby-Formel berechnete Wirkung | | |

**Patentansprüche**

1. Mittel enthaltend eine synergistisch wirksame Mischung aus Verbindungen der Formel (I)

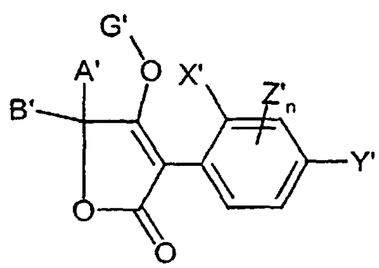

(I)

in welcher

X' für $C_1$-$C_6$-Alkyl, Halogen, $C_1$-$C_6$-Alkoxy oder $C_1$-$C_3$-Halogenalkyl steht,

Y' für Wasserstoff, $C_1$-$C_6$-Alkyl, Halogen, $C_1$-$C_6$-Alkoxy, $C_1$-$C_3$-Halogenalkyl steht,

Z' für $C_1$-$C_6$-Alkyl, Halogen, $C_1$-$C_6$-Alkoxy steht,

n für eine Zahl von 0-3 steht,

A' und B' gleich oder verschieden sind und für Wasserstoff oder gegebenenfalls durch Halogen substituiertes geradkettiges oder verzweigtes $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_2$-$C_8$-alkyl, $C_1$-$C_{10}$-Alkylthio-$C_2$-$C_8$-alkyl, Cycloalkyl mit 3-8 Ringatomen, das durch Sauerstoff und/oder Schwefel unterbrochen sein kann und gegebenenfalls durch Halogen, $C_1$-$C_6$-Alkyl, $C_1$-$C_6$-Halogenalkyl-, $C_1$-$C_6$-Alkoxy-, $C_1$-$C_6$-Halogenalkoxy, Nitro substituiertes Phenyl oder Phenyl-$C_1$-$C_6$-alkyl steht,

oder worin

A' und B' gemeinsam mit dem Kohlenstoffatom, an das sie gebunden sind einen gesättigten oder ungesättigten, gegebenenfalls durch Sauerstoff und/oder Schwefel unterbrochenen und gegebenenfalls durch Halogen, $C_1$-$C_6$-Alkyl, $C_1$-$C_6$-Alkoxy, $C_1$-$C_4$-Halogenalkyl, $C_1$-$C_4$-Halogenalkoxy, $C_1$-$C_4$-Alkylthio oder gegebenenfalls substituiertes Phenyl substituierten oder gegebenenfalls benzokondensierten 3- bis 8-gliedrigen Ring bilden,

G' für Wasserstoff (a) oder für die Gruppen

$-CO-R^1$ (b), 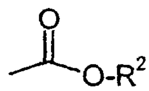 (c), $-SO_2-R^3$ (d),

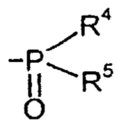 (e) oder 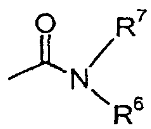 (f)

steht, in welchen

$R^1$ für gegebenenfalls durch Halogen substituiertes: $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Alkylthio-$C_2$-$C_8$-alkyl, $C_1$-$C_8$-Polyalkoxy-$C_2$-$C_8$-alkyl oder Cycloalkyl mit 3-8 Ringatomen, das durch Sauerstoff- und/oder Schwefelatome unterbrochen sein kann, steht, für gegebenenfalls durch Halogen, Nitro, $C_1$-$C_6$-Alkyl, $C_1$-$C_6$-Alkoxy, $C_1$-$C_6$-Halogenalkyl, $C_1$-$C_6$-Halogenalkoxy-substituiertes Phenyl steht;
für gegebenenfalls durch Halogen-, $C_1$-$C_6$-Alkyl, $C_1$-$C_6$-Alkoxy-, $C_1$-$C_6$-Halogenalkyl-, $C_1$-$C_6$-Halogenalkoxy-substituiertes Phenyl-$C_1$-$C_6$-alkyl steht,
für gegebenenfalls durch Halogen und/oder $C_1$-$C_6$-Alkyl substituiertes Pyridyl, Pyrimidyl, Thiazolyl und Pyrazolyl steht,
für gegebenenfalls durch Halogen und/oder $C_1$-$C_6$-Alkyl-substituiertes Phenoxy-$C_1$-$C_6$-alkyl steht,

$R^2$ für gegebenenfalls durch Halogen substituiertes: $C_1$-$C_{20}$-Alkyl, $C_2$-$C_{20}$-Alkenyl, $C_1$-$C_8$-Alkoxy-$C_2$-$C_8$-alkyl, $C_1$-$C_8$-Polyalkoxy-$C_2$-$C_8$-alkyl steht,
für gegebenenfalls durch Halogen, Nitro, $C_1$-$C_6$-Alkyl, $C_1$-$C_6$-Alkoxy, $C_1$-$C_6$-Halogenalkyl-substituiertes Phenyl oder Benzyl steht,

$R^3$, $R^4$ und $R^5$ unabhängig voneinander für gegebenenfalls durch Halogen substituiertes $C_1$-$C_8$-Alkyl, $C_1$-$C_8$-Alkoxy, $C_1$-$C_8$-Alkylamino, Di-($C_1$-$C_8$)-Alkylamino, $C_1$-$C_8$-Alkylthio, $C_2$-$C_5$-Alkenylthio, $C_2$-$C_5$-Alkinylthio, $C_3$-$C_7$-Cycloalkylthio, für gegebenenfalls durch Halogen, Nitro, Cyano, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkyl, $C_1$-$C_4$-Halogenalkyl substituiertes Phenyl, Phenoxy oder Phenylthio stehen,

$R^6$ und $R^7$ unabhängig voneinander für gegebenenfalls durch Halogen substituiertes $C_1$-$C_{20}$-Alkyl, $C_1$-$C_{20}$-

Alkoxy, $C_2$-$C_8$-Alkenyl, $C_1$-$C_{20}$-Alkoxy-$C_1$-$C_{20}$-alkyl, für gegebenenfalls durch Halogen, $C_1$-$C_{20}$-Halogenalkyl, $C_1$-$C_{20}$-Alkyl oder $C_1$-$C_{20}$-Alkoxy substituiertes Phenyl, für gegebenenfalls durch Halogen, $C_1$-$C_{20}$-Alkyl, $C_1$-$C_{20}$-Halogenalkyl oder $C_1$-$C_{20}$-Alkoxy substituiertes Benzyl steht oder zusammen für einen gegebenenfalls durch Sauerstoff unterbrochenen $C_2$-$C_6$-Alkylenring stehen,

und mindestens einen Agonisten bzw. Antagonisten von nicotinergen Acetylcholinrezeptoren.

**2.** Mittel enthaltend eine synergistisch wirksame Mischung aus Verbindungen der Formel (I) gemäß Anspruch 1, in welcher

X' für $C_1$-$C_4$-Alkyl, Halogen, $C_1$-$C_4$-Alkoxy oder $C_1$-$C_2$-Halogenalkyl steht,

Y' für Wasserstoff, $C_1$-$C_4$-Alkyl, Halogen, $C_1$-$C_4$-Alkoxy, $C_1$-$C_2$-Halogenalkyl steht,

Z' für $C_1$-$C_4$-Alkyl, Halogen, $C_1$-$C_4$-Alkoxy steht,

n für 0 oder 1 steht,

A' und B' gemeinsam mit dem Kohlenstoffatom, an das sie gebunden sind einen gesättigten gegebenenfalls durch $C_1$-$C_4$-Alkyl, $C_1$-$C_4$-Alkoxy, substituierten 5- bis 6-gliedrigen Ring bilden,

G' für Wasserstoff (a) oder für die Gruppen

$-CO-R^1$ (b) 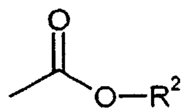 (c)

in welchen

$R^1$ für gegebenenfalls durch Halogen substituiertes: $C_1$-$C_{16}$-Alkyl, $C_2$-$C_{16}$-Alkenyl, $C_1$-$C_6$-Alkoxy-$C_2$-$C_6$-alkyl, oder Cycloalkyl mit 3-7 Ringatomen, das durch 1 bis 2 Sauerstoff- und/oder Schwefelatome unterbrochen sein kann, steht,
für gegebenenfalls durch Halogen, Nitro, $C_1$-$C_4$-Alkyl, $C_1$-$C_4$-Alkoxy, $C_1$-$C_3$-Halogenalkyl, $C_1$-$C_3$-Halogenalkoxy-substituiertes Phenyl steht;

$R^2$ für gegebenenfalls durch Halogen substituiertes: $C_1$-$C_{16}$-Alkyl, $C_2$-$C_{16}$-Alkenyl, $C_1$-$C_6$-Alkoxy-$C_2$-$C_6$-alkyl, steht,
für gegebenenfalls durch Halogen, Nitro, $C_1$-$C_4$-Alkyl, $C_1$-$C_4$-Alkoxy, $C_1$-$C_4$-Halogenalkyl-substituiertes Phenyl oder Benzyl steht,

und mindestens einen Agonisten bzw. Antagonisten von nicotinergen Acetylcholinrezeptoren.

**3.** Mittel enthaltend eine synergistisch wirksame Mischung aus der Verbindung der Formel (Ia)

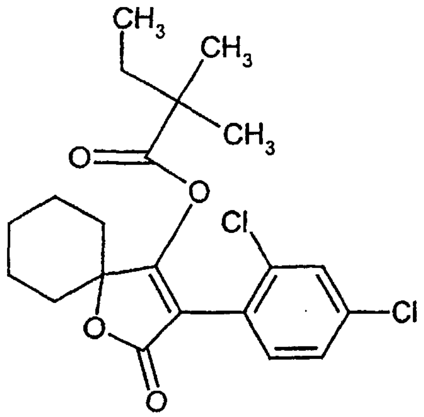

(Ia)

und mindestens einen Agonisten bzw. Antagonisten von nicotinergen Acetylcholinrezeptoren.

**4.** Mittel gemäß Anspruch 1, 2 oder 3 enthaltend Verbindungen der Formel (I) und den Agonisten bzw. Antagonisten von nicotinergen Acetylcholinrezeptoren im Verhältnis von 1:100 bis 100:1.

**5.** Verwendung einer synergistisch wirksamen Mischung, enthaltend Verbindungen der Formel (I) gemäß Anspruch 1, 2 oder 3 und mindestens einen Agonisten bzw. Antagonisten von nicotinergen Acetylcholinrezeptoren zur Bekämpfung von tierischen Schädlingen.

**6.** Verfahren zur Bekämpfung tierischer Schädlinge, **dadurch gekennzeichnet, dass** man Mischungen wie in Anspruch 1, 2 oder 3 definiert, auf tierische Schädlinge und/oder deren Lebensraum einwirken lässt.

**7.** Verfahren zur Herstellung von Schädlingsbekämpfungsmitteln, **dadurch gekennzeichnet, dass** man eine synergistisch wirksame Mischung, enthaltend Verbindungen der Formel (I) gemäß Anspruch 1, 2 oder 3 und mindestens einen Agonisten bzw. Antagonisten von nicotinergen Acetylcholinrezeptoren mit Streckmitteln und/oder oberflächenaktiven Substanzen vermischt.

**8.** Mischungen gemäß Anspruch 1, 2, 3 oder 4 mindestens eine der folgenden Verbindungen enthaltend

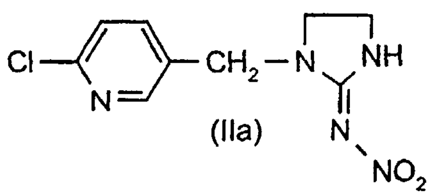

(IIa)

oder

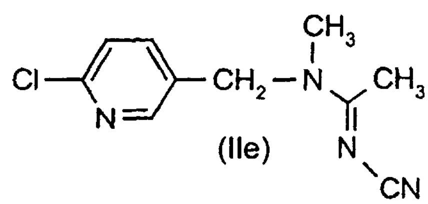

(IIe)

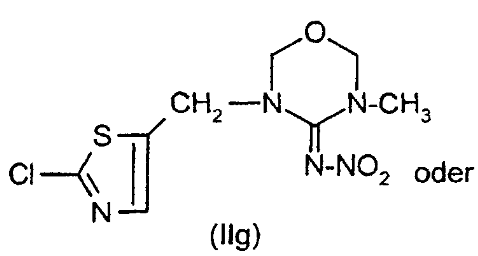

(IIg)

oder

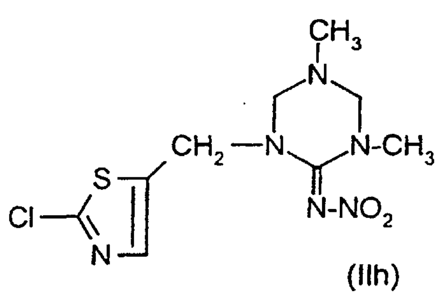

(IIh)

oder

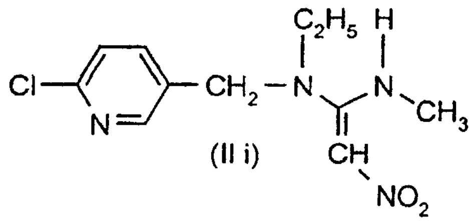

(II i)

oder

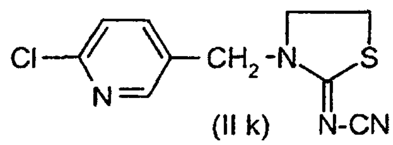

(II k)

oder

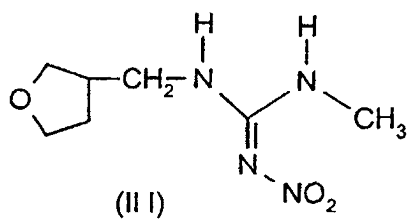

(II l)

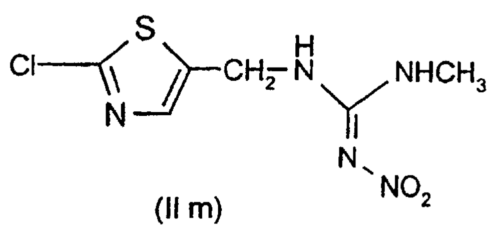

(II m)

## Claims

**1.** Composition, comprising a synergistically effective mixture of compounds of the formula (I)

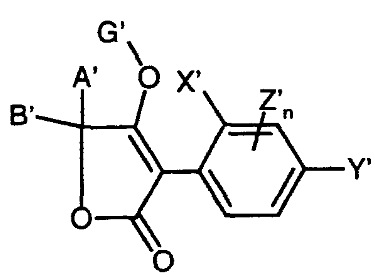

(I)

in which

X' represents $C_1$-$C_6$-alkyl, halogen, $C_1$-$C_6$-alkoxy or $C_1$-$C_3$-halogenoalkyl,

Y' represents hydrogen, $C_1$-$C_6$-alkyl, halogen, $C_1$-$C_6$-alkoxy, $C_1$-$C_3$-halogenoalkyl,

Z' represents $C_1$-$C_6$-alkyl, halogen, $C_1$-$C_6$-alkoxy,

n represents a number from 0 to 3,

A' and B' are identical or different and each represents hydrogen or in each case optionally halogen-substituted straight-chain or branched $C_1$-$C_{12}$-alkyl, $C_3$-$C_8$-alkenyl, $C_3$-$C_8$-alkinyl, $C_1$-$C_{10}$-alkoxy-$C_2$-$C_8$-alkyl, $C_1$-$C_8$-polyalkoxy-$C_2$-$C_8$-alkyl, $C_1$-$C_{10}$-alkylthio-$C_2$-$C_8$-alkyl, cycloalkyl having 3-8 ring atoms which may be interrupted by oxygen and/or sulphur and in each case optionally halogen-, $C_1$-$C_6$-alkyl-, $C_1$-$C_6$-halogenoalkyl-, $C_1$-$C_6$-alkoxy-, $C_1$-$C_6$-halogenoalkoxy- and/or nitro-substituted phenyl or phenyl-$C_1$-$C_6$-alkyl,

or in which

A' and B' together with the carbon atom to which they are attached form a saturated or unsaturated 3- to 8-membered ring which is optionally interrupted by oxygen and/or sulphur and is optionally substituted by halogen, $C_1$-$C_6$-alkyl, $C_1$-$C_6$-alkoxy, $C_1$-$C_4$-halogenoalkyl, $C_1$-$C_4$-halogenoalkoxy, $C_1$-$C_4$-alkylthio or optionally substituted phenyl or is optionally benzo-fused,

G' represents hydrogen (a) or represents the groups

$-CO-R^1$ (b), 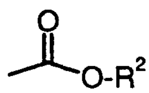 (c), $-SO_2-R^3$ (d),

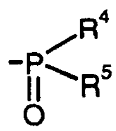 (e) or 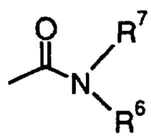 (f)

in which

$R^1$ represents in each case optionally halogen-substituted $C_1$-$C_{20}$-alkyl, $C_2$-$C_{20}$-alkenyl, $C_1$-$C_8$-alkoxy-$C_2$-$C_8$-alkyl, $C_1$-$C_8$-alkylthio-$C_2$-$C_8$-alkyl, $C_1$-$C_8$-polyalkoxy-$C_2$-$C_8$-alkyl or cycloalkyl having 3-8 ring members which may be interrupted by oxygen and/or sulphur atoms,
represents optionally halogen-, nitro-, $C_1$-$C_6$-alkyl-, $C_1$-$C_6$-alkoxy-, $C_1$-$C_6$-halogenoalkyl-and/or $C_1$-$C_6$-halogenoalkoxy-substituted phenyl;
represents optionally halogen-, $C_1$-$C_6$-alkyl-, $C_1$-$C_6$-alkoxy-, $C_1$-$C_6$-halogenoalkyl- and/or $C_1$-$C_6$-halogenoalkoxy-substituted phenyl-$C_1$-$C_6$-alkyl,
represents in each case optionally halogen- and/or $C_1$-$C_6$-alkyl-substituted pyridyl, pyrimidyl, thiazolyl and pyrazolyl,
or represents optionally halogen- and/or $C_1$-$C_6$-alkyl-substituted phenoxy-$C_1$-$C_6$-alkyl,

$R^2$ represents in each case optionally halogen-substituted $C_1$-$C_{20}$-alkyl, $C_2$-$C_{20}$-alkenyl, $C_1$-$C_8$-alkoxy-$C_2$-$C_8$-alkyl, $C_1$-$C_8$-polyalkoxy-$C_2$-$C_8$-alkyl,
represents in each case optionally halogen-, nitro-, $C_1$-$C_6$-alkyl, $C_1$-$C_6$-alkoxy- and/or $C_1$-$C_6$-halogenoalkyl-substituted phenyl or benzyl,

$R^3$, $R^4$ and $R^5$ independently of one another each represent in each case optionally halogen-substituted $C_1$-$C_8$-alkyl, $C_1$-$C_8$-alkoxy, $C_1$-$C_8$-alkylamino, di-($C_1$-$C_8$)-alkylamino, $C_1$-$C_8$-alkylthio, $C_2$-$C_5$-alkenylthio, $C_2$-$C_5$-alkinyl-thio, $C_3$-$C_7$-cycloalkylthio, represent in each case optionally halogen-, nitro-, cyano-, $C_1$-$C_4$-alkoxy-, $C_1$-$C_4$-halogenoalkoxy-, $C_1$-$C_4$-alkylthio-, $C_1$-$C_4$-halogenoalkylthio-, $C_1$-$C_4$-alkyl- and/or $C_1$-$C_4$-halogenoalkyl-substituted phenyl, phenoxy or phenylthio,

$R^6$ and $R^7$ independently of one another each represent in each case optionally halogen-substituted $C_1$-$C_{20}$-alkyl, $C_1$-$C_{20}$-alkoxy, $C_2$-$C_8$-alkenyl, $C_1$-$C_{20}$-alkoxy-$C_1$-$C_{20}$-alkyl, represent in each case optionally halogen-, $C_1$-$C_{20}$-halogenoalkyl-, $C_1$-$C_{20}$-alkyl-or $C_1$-$C_{20}$-alkoxy-substituted phenyl, represent in each case optionally halogen-, $C_1$-$C_{20}$-alkyl-, $C_1$-$C_{20}$-halogenoalkyl- or $C_1$-$C_{20}$-alkoxy-substituted benzyl or together represent a $C_2$-$C_6$-alkylene ring which is optionally interrupted by oxygen,

and at least one agonist or antagonist of nicotinic acetylcholine receptors.

2. Composition, comprising a synergistically effective mixture of compounds of the formula (I) according to Claim 1, in which

X' represents $C_1$-$C_4$-alkyl, halogen, $C_1$-$C_4$-alkoxy or $C_1$-$C_2$-halogenoalkyl,

Y' represents hydrogen, $C_1$-$C_4$-alkyl, halogen, $C_1$-$C_4$-alkoxy, $C_1$-$C_2$-halogenoalkyl,

Z' represents $C_1$-$C_4$-alkyl, halogen, $C_1$-$C_4$-alkoxy,

n represents 0 or 1,

A' and B' together with the carbon atom to which they are attached form a saturated 5- to 6-membered ring which is optionally substituted by $C_1$-$C_4$-alkyl and/or $C_1$-$C_4$-alkoxy,

G' represents hydrogen (a) or represents the groups

$—CO—R^1$ (b) 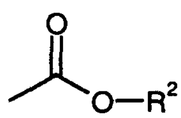 (c)

in which

$R^1$ represents in each case optionally halogen-substituted $C_1$-$C_{16}$-alkyl, $C_2$-$C_{16}$-alkenyl, $C_1$-$C_6$-alkoxy-$C_2$-$C_6$-alkyl or cycloalkyl having 3-7 ring atoms which may be interrupted by 1 to 2 oxygen and/or sulphur atoms,
represents optionally halogen-, nitro-, $C_1$-$C_4$-alkyl-, $C_1$-$C_4$-alkoxy-, $C_1$-$C_3$-halogenoalkyl-and/or $C_1$-$C_3$-halogenoalkoxy-substituted phenyl;

$R^2$ represents in each case optionally halogen-substituted $C_1$-$C_{16}$-alkyl, $C_2$-$C_{16}$-alkenyl or $C_1$-$C_6$-alkoxy-$C_2$-$C_6$-alkyl,
represents in each optionally halogen-, nitro-, $C_1$-$C_4$-alkyl-, $C_1$-$C_4$-alkoxy- and/or $C_1$-$C_4$-halogenoalkyl-substituted phenyl or ben-zyl,

and at least one agonist or antagonist of nicotinic acetylcholine receptors.

3. Composition, comprising a synergistically effective mixture of the compound of the formula (Ia)

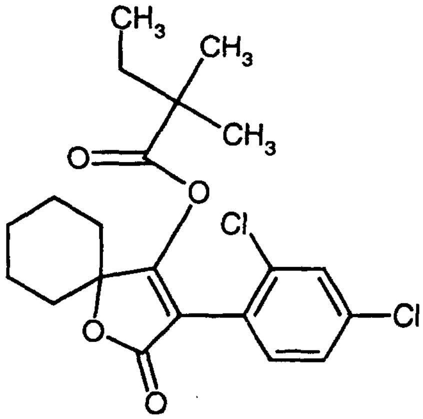

(Ia)

and at least one agonist or antagonist of nicotinic acetylcholine receptors.

**4.** Composition according to any of Claims 1, 2 and 3, comprising compounds of the formula (I) and the agonist or antagonist of nicotinic acetylcholine receptors in a ratio of from 1:100 to 100:1.

**5.** Use of a synergistically effective mixture, comprising compounds of the formula (I) according to any of Claims 1, 2 and 3, and at least one agonist or antagonist of nicotinic acetylcholine receptors, for controlling animal pests.

**6.** Method for controlling animal pests, **characterized in that** mixtures as defined in any of Claims 1, 2 and 3 are allowed to act on animal pests and/or their habitat.

**7.** Process for preparing pesticides, **characterized in that** a synergistially effective amount comprising compounds of the formula (I) according to any of Claims 1, 2 and 3 and at least one agonist or antagonist of nicotinic acetylcholine receptors is mixed with extenders and/or surfactants.

**8.** Mixtures according to any of Claims 1, 2, 3 and 4, comprising at least one of the following compounds

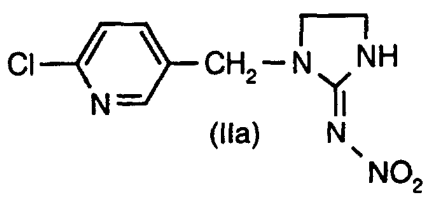

(IIa) or

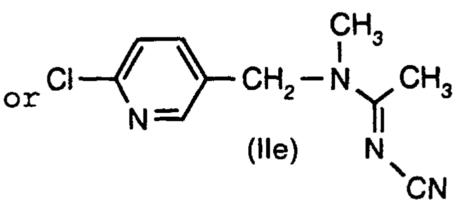

(IIe)

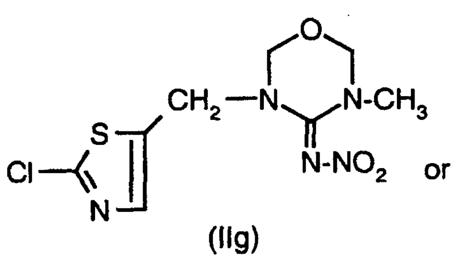

(IIg) or

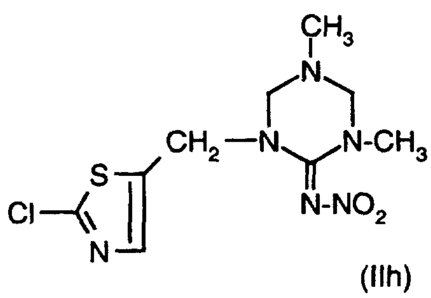

(IIh)

or

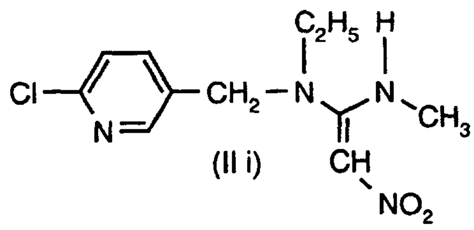

(II i)

or

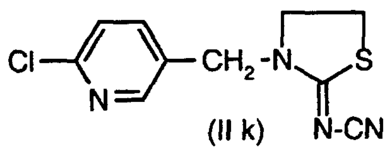

(II k)

or

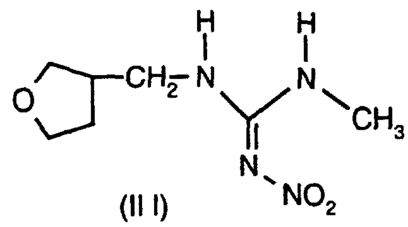

(II l)

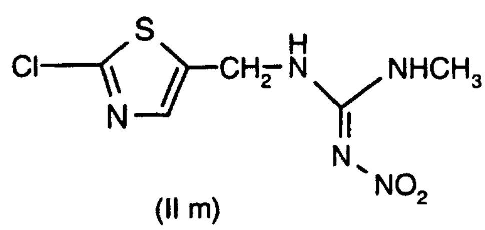

(II m)

## Revendications

**1.** Compositions contenant un mélange à effet synergique de composés de formule (I)

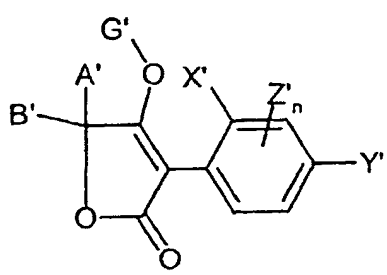

(I)

dans laquelle

X' est un reste alkyle en $C_1$ à $C_6$, un halogène, un reste alkoxy en $C_1$ à $C_6$ ou un reste halogénalkyle en $C_1$ à $C_3$,

Y' est l'hydrogène, un reste alkyle en $C_1$ à $C_6$, un halogène, un reste alkoxy en $C_1$ à $C_6$, halogénalkyle en $C_1$ à $C_3$,

Z' est un reste alkyle en $C_1$ à $C_6$, un halogène, un reste alkoxy en $C_1$ à $C_6$,

n représente un nombre de 0 à 3,

A' et B' sont identiques ou différents et représentent l'hydrogène ou un reste éventuellement substitué par un halogène, linéaire ou ramifié, alkyle en $C_1$ à $C_{12}$, alcényle en $C_3$ à $C_8$, alcynyle en $C_3$ à $C_8$, (alkoxy en $C_1$ à $C_{10}$)-(alkyle en $C_2$ à $C_8$), poly(alkoxy en $C_1$ à $C_8$)-(alkyle en $C_2$ à $C_8$), (alkylthio en $C_1$ à $C_{10}$)-(alkyle en $C_2$ à $C_8$), cycloalkyle à noyau de 3 à 8 atomes, qui peut être interrompu par de l'oxygène et/ou du soufre, et phényle ou phényl-(alkyle en $C_1$ à $C_6$) éventuellement substitué par un halogène, un radical alkyle en $C_1$ à $C_6$, halogénalkyle en $C_1$ à $C_6$, alkoxy en $C_1$ à $C_6$, halogénalkoxy en $C_1$ à $C_6$, nitro,

ou dans laquelle

A' et B' forment conjointement avec l'atome de carbone auquel ils sont liés un noyau de 3 à 8 chaînons saturé ou non saturé, éventuellement interrompu par de l'oxygène et/ou du soufre et éventuellement substitué par un halogène, un radical alkyle en $C_1$ à $C_6$, alkoxy en $C_1$ à $C_6$, halogénalkyle en $C_1$ à $C_4$, halogénalkoxy en $C_1$ à $C_4$, alkylthio en $C_1$ à $C_4$ ou phényle éventuellement substitué,

G' représente l'hydrogène (a) ou les groupes

$-CO-R^1$ (b), 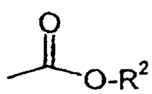 (c), $-SO_2-R^3$ (d)

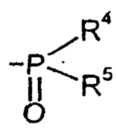 (e) ou 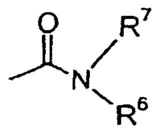 (f)

dans lesquels

$R^1$ est un reste, éventuellement substitué par un halogène : alkyle en $C_1$ à $C_{20}$, alcényle en $C_2$ à $C_{20}$, (alkoxy en $C_1$ à $C_8$)-(alkyle en $C_2$ à $C_8$), (alkylthio en $C_1$ à $C_8$)-(alkyle en $C_2$ à $C_8$), poly(alkoxy en $C_1$ à $C_8$)-(alkyle en $C_2$ à $C_8$) ou cycloalkyle à noyau de 3 à 8 atomes, qui peut être interrompu par des atomes d'oxygène et/ou de soufre,
un reste phényle éventuellement substitué par un radical halogéno, nitro, alkyle en $C_1$ à $C_6$, alkoxy en $C_1$ à $C_6$, halogénalkyle en $C_1$ à $C_6$, halogénalkoxy en $C_1$ à $C_6$ ;
un reste phényl-(alkyle en $C_1$ à $C_6$) éventuellement substitué par un radical halogéno, alkyle en $C_1$ à $C_6$, alkoxy en $C_1$ à $C_6$, halogénalkyle en $C_1$ à $C_6$, halogén-alkoxy en $C_1$ à $C_6$,
un reste pyridyle, pyrimidyle, thiazolyle ou pyrazolyle éventuellement substitué par un radical halogéno et/ou alkyle en $C_1$ à $C_6$,
un reste phénoxy-(alkyle en $C_1$ à $C_6$) éventuellement substitué par un halogène et/ou un radical alkyle en $C_1$ à $C_6$,

$R^2$ est un reste, éventuellement substitué par un halogène : alkyle en $C_1$ à $C_{20}$, alcényle en $C_2$ à $C_{20}$, (alkoxy en $C_1$ à $C_8$)-(alkyle en $C_2$ à $C_8$), poly(alkoxy en $C_1$ à $C_8$)-(alkyle en $C_2$ à $C_8$),
un reste phényle ou benzyle éventuellement substitué par un radical halogéno, nitro, alkyle en $C_1$ à $C_6$, alkoxy en $C_1$ à $C_6$, halogénalkyle en $C_1$ à $C_6$,

$R^3$, $R^4$ et $R^5$ représentent, indépendamment l'un de l'autre, un radical, éventuellement substitué par un halogène, alkyle en $C_1$ à $C_8$, alkoxy en $C_1$ à $C_8$, alkylamino en $C_1$ à $C_8$, di(alkyle en $C_1$ à $C_8$)amino, alkylthio en $C_1$ à $C_8$, alcénylthio en $C_2$ à $C_5$, alcynylthio en $C_2$ à $C_5$, cycloalkylthio en $C_3$ à $C_7$, un

reste phényle, phénoxy ou phénylthio portant éventuellement un substituant halogéno, nitro, cyano, alkoxy en $C_1$ à $C_4$, halogén-alkoxy en $C_1$ à $C_4$, alkylthio en $C_1$ à $C_4$, halogénalkyl-thio en $C_1$ à $C_4$, alkyle en $C_1$ à $C_4$, halogénalkyle en $C_1$ à $C_4$,

$R^6$ et $R^7$ représentent, indépendamment l'un de l'autre, un reste, éventuellement substitué par un halogène, alkyle en $C_1$ à $C_{20}$, alkoxy en $C_1$ à $C_{20}$, alcényle en $C_2$ à $C_8$, (alkoxy en $C_1$ à $C_{20}$)-(alkyle en $C_1$ à $C_{20}$), un reste phényle éventuellement substitué par un radical halogéno, halogénalkyle en $C_1$ à $C_{20}$, alkyle en $C_1$ à $C_{20}$ ou alkoxy en $C_1$ à $C_{20}$, un reste benzyle éventuellement substitué par un radical halogéno, alkyle en $C_1$ à $C_{20}$, halogénalkyle en $C_1$ à $C_{20}$ ou alkoxy en $C_1$ à $C_{20}$, ou forment ensemble un noyau alkylène en $C_2$ à $C_6$ éventuellement interrompu par de l'oxygène,

et au moins un agoniste ou un antagoniste des récepteurs nicotinergiques d'acétylcholine.

**2.** Compositions contenant un mélange à effet synergique de composés de formule (I) suivant la revendication 1, dans laquelle

X' est un reste alkyle en $C_1$ à $C_4$, un halogène, un reste alkoxy en $C_1$ à $C_4$ ou halogénalkyle en $C_1$ ou $C_2$,

Y' est l'hydrogène, un reste alkyle en $C_1$ à $C_4$, un halogène, un reste alkoxy en $C_1$ à $C_4$ ou halogénalkyle en $C_1$ ou $C_2$,

Z' est un reste alkyle en $C_1$ à $C_4$, un halogène, un reste alkoxy en $C_1$ à $C_4$,

n a la valeur 0 ou 1,

A' et B' forment conjointement avec l'atome de carbone auquel ils sont liés un noyau pentagonal ou hexagonal éventuellement substitué par un radical alkyle en $C_1$ à $C_4$ ou alkoxy en $C_1$ à $C_4$,

G' représente l'hydrogène (a) ou les groupes

$-CO-R^1$ (b) 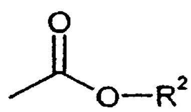 (c)

dans lesquels

$R^1$ est un reste, éventuellement substitué par un halogène : alkyle en $C_1$ à $C_{16}$, alcényle en $C_2$ à $C_{16}$, (alkoxy en $C_1$ à $C_6$)-(alkyle en $C_2$ à $C_6$) ou cycloalkyle à noyau de 3 à 7 atomes, qui est interrompu par 1 ou 2 atomes d'oxygène et/ou de soufre,
un reste phényle éventuellement substitué par un radical halogéno, nitro, alkyle en $C_1$ à $C_4$, alkoxy en $C_1$ à $C_4$, halogénalkyle en $C_1$ à $C_3$, halogénalkoxy en $C_1$ à $C_3$ ;

$R^2$ représente un reste, éventuellement substitué par un halogène : alkyle en $C_1$ à $C_{16}$, alcényle en $C_2$ à $C_{16}$, (alkoxy en $C_1$ à $C_6$)-(alkyle en $C_2$ à $C_6$),
un reste phényle ou benzyle éventuellement substitué par un radical halogéno, nitro, alkyle en $C_1$ à $C_4$, alkoxy en $C_1$ à $C_4$, halogénalkyle en $C_1$ à $C_4$,

et au moins un agoniste ou un antagoniste de récepteurs nicotinergiques d'acétylcholine.

**3.** Compositions contenant un mélange à effet synergique du composé de formule (Ia)

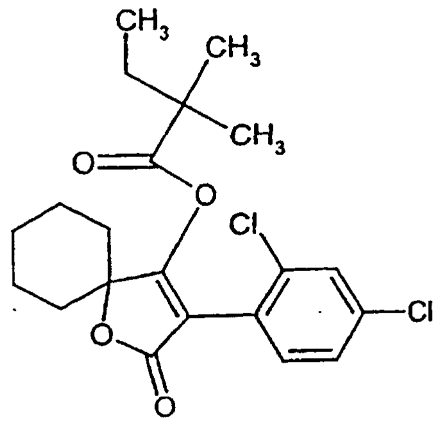

(Ia)

et au moins un agoniste ou un antagoniste de récepteurs nicotinergiques d'acétylcholine.

4. Compositions suivant la revendication 1, 2 ou 3, contenant des composés de formule (I) et l'agoniste ou l'antagoniste de récepteurs nicotinergiques d'acétylcholine dans un rapport de 1:100 à 100:1.

5. Utilisation d'un mélange à effet synergique contenant des composés de formule (I) suivant la revendication 1, 2 ou 3 et au moins un agoniste ou antagoniste de récepteurs nicotinergiques d'acétylcholine, pour la lutte contre des parasites animaux.

6. Procédé de lutte contre des parasites animaux, **caractérisé en ce qu'**on fait agir sur des parasites animaux et/ou sur leur milieu des mélanges tels que définis dans la revendication 1, 2 ou 3.

7. Procédé de préparation de compositions pour la lutte antiparasitaire, **caractérisé en ce qu'**on mélange avec des diluants et/ou des substances tensio-actives un mélange à effet synergique contenant des composés de formule (I) suivant la revendication 1, 2 ou 3 et au moins un agoniste ou antagoniste de récepteurs nicotinergiques d'acétylcholine.

8. Mélanges suivant la revendication 1, 2, 3 ou 4, contenant au moins l'un des composés suivants :

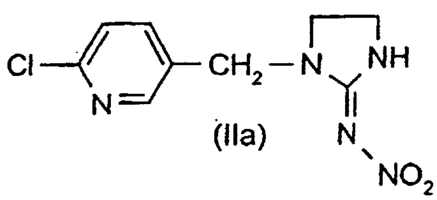

(IIa)

ou

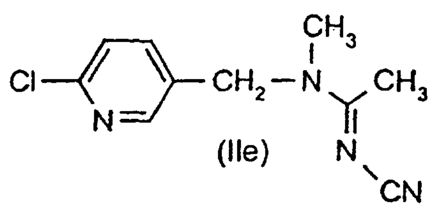

(IIe)

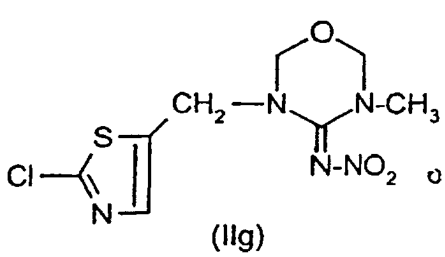

ou

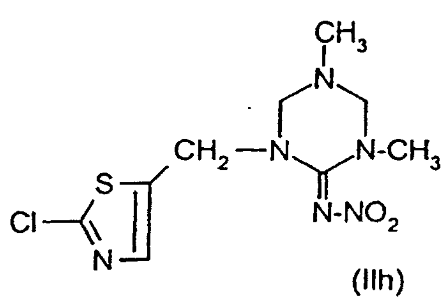

(IIg) (IIh)

ou

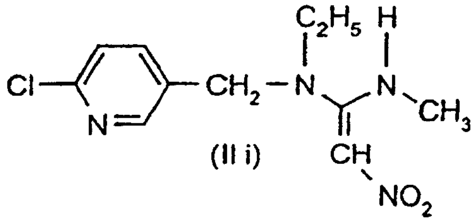

(II i)

ou

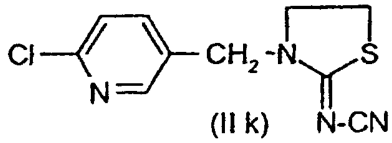

(II k)

ou

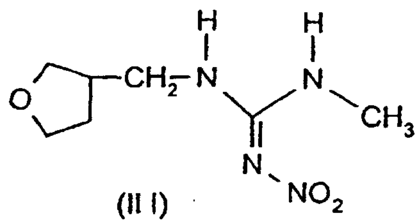

(II l)

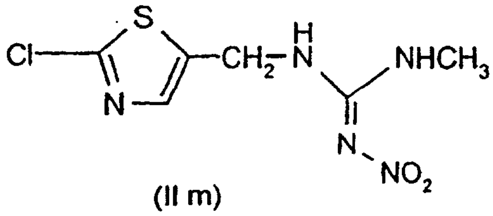

(II m)